Europäisches Patentamt

European Patent Office

Office européen des brevets

(19)

(11) EP 1 538 196 A1

## (12) EUROPEAN PATENT APPLICATION
published in accordance with Art. 158(3) EPC

(43) Date of publication:
08.06.2005 Bulletin 2005/23

(51) Int Cl.⁷: $C12M\ 3/00$, $A61L\ 27/22$, $C12N\ 5/06$, $C12N\ 11/02$

(21) Application number: 03792825.6

(22) Date of filing: 25.08.2003

(86) International application number:
PCT/JP2003/010692

(87) International publication number:
WO 2004/018615 (04.03.2004 Gazette 2004/10)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 23.08.2002 JP 2002243923
23.08.2002 JP 2002244294

(71) Applicant: ASAHI MEDICAL Co., Ltd.
Tokyo 101-8482 (JP)

(72) Inventors:
• TOKUSHIMA, Yasuo
Fuji-shi, Shizuoka 416-0931 (JP)

• KITAGUCHI, Nobuya
Fuji-shi, Shizuoka 417-0001 (JP)
• INADOME, Shuichiro
Oita-shi, Oita 870-0921 (JP)
• HORI, Takahiro
Oita-shi, Oita 870-0836 (JP)

(74) Representative:
von Kreisler, Alek, Dipl.-Chem. et al
Deichmannhaus am Dom,
Postfach 10 22 41
50462 Köln (DE)

### (54) FIBRIN-CONTAINING COMPOSITION

(57) It is an object of the present invention to provide a scaffold with high performance, which is suitable for cell growth and differentiation in regenerative medicine. The present invention provides a fibrin-containing biological scaffold, which is **characterized in that** it comprises a mixture consisting of a fibrinogen concentrate obtained from human plasma by a short-time rough purification step and a fibrinogen activator, when a fibrin composition is used in the regeneration of human tissues and the cell growth.

EP 1 538 196 A1

**Description**

TECHNICAL FIELD

The present invention relates to a biological scaffold for tissue regeneration, a production method thereof, and a production system thereof.

BACKGROUND ART

In recent years, the use of regenerative medicine has been greatly anticipated instead of conventional organ transplantation or therapeutic methods using artificial organs. Studies regarding regenerative medicine have been developed in two directions: studies directed towards regeneration of cells or tissues by replenishment of stem cells, such as bone marrow transplantation, wound healing, and repair of the skin or organs damaged by surgical operations; and a series of studies that are narrowly interpreted as tissue engineering. Such tissue engineering in a narrow sense involves studies for repairing tissues using cells and a scaffold necessary for engraftment of the cells *in vivo*. Regenerative medicine requires "cells," a "scaffold used for the growth and differentiation of cells," and a "cell growth factor." For materials of such scaffolds, both biocompatibility, whereby the materials do not harm a living body, and bioabsorption, whereby the materials are decomposed and absorbed in the living body when new living tissues are formed, are required. Thus, biological materials are required for scaffolds.

Fibrin glue is a glue for external use by utilizing physiological blood coagulation reaction, which enables the adhesion and sealing of tissues and the subsequent wound healing. At present, such fibrin glue is broadly used in various types of surgical operations. In addition, a large number of techniques of using such fibrin glue as a biological scaffold in regenerative medicine have been known (Cell Transplantation, 7, 309-317, 1998; and Burn, 24, 621-630, 1998).

In order to prevent side effects including immune reactions such as allergic reactions or shock, cryoprecipitate (Cryo) prepared from autologous plasma has conventionally been used for fibrin glue. Needless to say, autologous fibrin glue prepared from autologous plasma can be used not only in tissue regeneration but also for hemostasis via adhesion and sealing of the patient's own tissues utilizing blood coagulation action. The following methods have been known as a matter of producing autologous fibrin glue from the blood.

For example, in the method of Casali et al., the plasma is frozen for a long period of time, thawed at a slow speed, and then centrifugation is carried out. It takes approximately 30 hours for the preparation of fibrin glue from the whole blood (Transmission, 32, 641-643, 1992; hereinafter this method is referred to as a "cryo method" in the present specification.).

A brief explanation of this method will be given below. First, the whole blood is collected, and is then centrifuged (1,000 g x 15 minutes, 4°C) to obtain plasma components. Thereafter, the plasma components obtained by centrifugation are transferred to an ultra-low temperature freezer, and the components are left at rest at -80°C for 18 hours, so that they become frozen. Thereafter, the plasma components that are in a frozen state are placed in a refrigerator at about 4°C, and they are thawed at a slow speed over 16 hours. When such freezing and slow-speed thawing treatments are carried out on the plasma components, a cryoprecipitate (fibrinogen, factor XIII) is precipitated in the plasma components. Thereafter, it is centrifuged at 1,000 x g for about 15 minutes using a refrigerated centrifuge (4°C). After thawing, it is further centrifuged at 4°C at 1,000 x g for about 15 minutes. The precipitate is recovered, and it is defined as a fibrinogen concentrate. There may also be cases where freezing and thawing are repeated under the aforementioned conditions after completion of the first thawing, while care is taken not to disturb the precipitate obtained as a result of the concentration (double cryo method).

There is another method described by Kjaergard using ethanol precipitation (Surg Gynecol Obstet, 175(1), July, 1992; hereinafter this method is referred to as an "ethanol method" in the present specification). This method comprises adding ethanol to the plasma obtained from the whole blood on ice water, so as to precipitate fibrinogen.

It has been reported that fibrin glue produced from fibrinogen obtained by the aforementioned cryo method or ethanol method can be used as a biological scaffold for regenerative medicine and the like. Examples of a technique of using fibrin glue as a biological scaffold may include: a technique of embedding fibroblasts in a fibrin gel that is prepared from fibrinogen obtained by the ethanol method, and seeding epithelial cells thereon, so as to produce artificial skin (Japanese Patent Application Laid-Open No. 10-277143); a technique of preparing a biological scaffold from a fibrin gel prepared from fibrinogen obtained by the ethanol method, and producing a keratinocyte culture product thereon, so as to produce an epidermal cell layer used for transplantation; and a technique of producing a dermic cell layer used for transplantation from fibroblasts and keratinocytes (Japanese Patent No. 3134079). Moreover, fibrins have been cultured using fibrinogens obtained by the cryo method and the ethanol method, and the obtained fibrins were compared in terms of the cell growth and metabolic function of chondrocytes. As a result, it was found that the cryo method was supported more strongly than the ethanol method in terms of the maintenance of phenotype, cell growth and proteoglycan accumulation of the chondrocytes (American Journal of Veterinary Research, 59, 514-520, 1998).

An improved cryo method that involves short-time freezing and rapid thawing to obtain fibrinogen for a short period of time of 1 to 2 hours has been known (National Publication of International Patent Application No. 2001-513073; hereinafter this method is referred to as an "improved cryo method" in the present specification). It has been reported that allogenic corneal epithelial stem cells are cultured in a fibrin gel that is produced from plasma as a material by the aforementioned improved cryo method, so as to produce a ocular surface tissue replacement product (cornea replacement) of the eye (Cornea 21(5): 505-510, 2002). In this report, however, the cells and the fibrin scaffold are not derived from the same individual, and the performance as a scaffold is not compared with that of another scaffold obtained by another method.

Regarding a method for producing fibrinogen used in preparation of autologous fibrin glue, a device for performing centrifugation after precipitation with an organic solvent, or for performing centrifugation after repeating freezing and thawing 8 to 10 times, has been disclosed (US Patent No. 6,083,383).

A method using ultrafiltration for concentration of fibrinogen has been proposed as a method for rapidly preparing autologous fibrin glue (National Publication of International Patent Application No. 11-508813). In this method, in order to concentrate a blood fraction, a "device comprising: an ultrafiltration unit having an outlet suitable for connecting to a vacuum source and first and second openings; a first valve for connecting the first opening of said ultrafiltration unit to a fluid delivery system for supplying the above blood fraction to said ultrafiltration unit; and a second valve for connecting the second opening of said ultrafiltration unit to a purge fluid delivery system" is used.

However, this method has been problematic in that: (1) introduction of plasma into the device cannot be conducted in a closed system; (2) a means for collecting plasma is required separately; and (3) the molecular weight cut off is 30,000 daltons, and thus, a low molecular weight protein fraction, such as albumin in plasma, is not permeable. As a result, a sufficient water-permeable effect cannot be obtained, and thus, fibrinogen cannot be highly concentrated. Consequently, a long coagulation time is required when fibrin glue is produced. Thus, only unstable products can be obtained. Moreover, this method has also been problematic in that: (4) since the concentration rate is not sufficient, and unless the concentrated plasma is further concentrated, the optimal fibrinogen concentration for the production of fibrin glue cannot be obtained; and (5) since the molecular weight cut off is 30,000 daltons, prothrombin cannot be separated from plasma containing concentrated fibrinogen, and thus, the produced fibrin glue is likely to be coagulated during the conservation period.

DISCLOSURE OF THE INVENTION

It is an object of the present invention to provide a scaffold with high performance, which is suitable for cell growth and differentiation in regenerative medicine. It is another object of the present invention to provide a method for rapidly and simply producing a safe, stable fibrin concentrate having no risk of infection, and a production system thereof.

As a result of intensive studies directed towards achieving the aforementioned objects, the present inventors have found that a fibrinogen concentrate obtained by a method involving short-time cooling and rapid thawing (improved cryo method) and a fibrinogen concentrate obtained using a plasma separation membrane having a specific cutoff value may be used as excellent scaffolds in regenerative medicine. Moreover, they have conceived of a fibrinogen concentration system comprising a plasma separation membrane that is a hollow fiber membrane, thereby completing the present invention.

Thus, the present invention provides the followings:

(1) A fibrin-containing biological scaffold, which is characterized in that it comprises a mixture consisting of a fibrinogen concentrate obtained from human plasma by a short-time rough purification step and a fibrinogen activator, when a fibrin composition is used in the regeneration of human tissues and the cell growth.

(2) The fibrin-containing biological scaffold according to (1) above, wherein said fibrinogen concentrate is obtained by a method comprising steps of cooling the plasma for a short time, rapidly thawing the plasma, and recovering the fibrinogen concentrate.

(3) The fibrin-containing biological scaffold according to (1) or (2) above, wherein said fibrinogen concentrate is obtained from the plasma by precipitation, resulting in a recovery rate of fibrinogen within the range between 15% and 32%.

(4) The fibrin-containing biological scaffold according to any one of (1) to (3) above, wherein said fibrinogen concentrate is obtained by a method comprising steps of cooling the plasma for 10 to 60 minutes, thawing the plasma for 15 to 60 minutes, and recovering the fibrinogen concentrate.

(5) The fibrin-containing biological scaffold according to (3) or (4) above, wherein the cooling step is carried out at a temperature between -20°C and -40°C and the thawing step is carried out at a temperature between -10°C and +15°C.

(6) The fibrin-containing biological scaffold according to (1) above, wherein said fibrinogen concentrate is obtained from human plasma using a plasma component fractionation device.

(7) The fibrin-containing biological scaffold according to (6) above, wherein said plasma component fractionation device comprises therein a hollow fiber membrane used for fractionation of plasma components.

(8) The fibrin-containing biological scaffold according to (7) above, wherein the material for said hollow fiber membrane is any one selected from the group consisting of hydrophilic polysulfone, EVAL (ethylene-vinyl alcohol copolymer), PAN (polyacrylonitrile), CDA (cellulose diacetate), and CTA (cellulose triacetate).

(9) The fibrin-containing biological scaffold according to (8) above, wherein said material for the hollow fiber membrane is hydrophilic polysulfone or EVAL (ethylene-vinyl alcohol copolymer).

(10) The fibrin-containing biological scaffold according to any one of (6) to (9) above, wherein the cutoff value of said hollow fiber membrane is between 80,000 daltons and 300,000 daltons.

(11) The fibrin-containing biological scaffold according to any one of (6) to (9) above, wherein the cutoff value of said hollow fiber membrane is between 150,000 daltons and 400,000 daltons.

(12) The fibrin-containing biological scaffold according to any one of (6) to (11) above, wherein said fibrinogen concentrate is obtained from the end of a hollow fiber by supplying human plasma to a hollow portion of the hollow fiber in a plasma component fractionation device, and allowing mainly liquid components to permeate from the inner surface of the hollow fiber to the outer surface thereof.

(13) The fibrin-containing biological scaffold according to (12) above, wherein the ratio (Bin/Bout) between the amount (Bin) of the patient plasma supplied to said hollow portion per unit time and the amount (Bout) of fibrinogen concentrate collected from the end of the hollow fiber per unit time is within the range between 2 and 20.

(14) The fibrin-containing biological scaffold according to (12) or (13) above, wherein said Bin/Bout ratio is within the range between 5 and 10.

(15) The fibrin-containing biological scaffold according to any one of (6) to (14) above, wherein said fibrinogen concentrate is obtained by allowing human plasma to come into contact with the outer surface of a hollow fiber in a plasma component fractionation device, and allowing mainly liquid components to permeate from the outer surface of the hollow fiber to the inner surface thereof, thereby concentrating the plasma allowed to be contacted with the outer portion of the hollow fiber.

(16) The fibrin-containing biological scaffold according to (15) above, wherein, when liquid components are allowed to permeate, the hollow portion of the hollow fiber is depressurized, and the liquid components are allowed to permeate by aspiration from the outer portion of the hollow fiber to the inner portion thereof.

(17) The fibrin-containing biological scaffold according to (15) or (16) above, wherein the ratio (Cinitial/Cend) between the amount (Cinitial) of plasma that is allowed to come into contact with the outer surface of said hollow fiber and the amount (Cend) of a fibrinogen concentrate obtained by allowing mainly liquid components to permeate and by concentrating the plasma that is allowed to be contacted with the outer surface of the hollow fiber is within the range between 2 and 20.

(18) The fibrin-containing biological scaffold according to any one of (15) to (17) above, wherein said ratio Cinitial/Cend is within the range between 5 and 10.

(19) The fibrin-containing biological scaffold according to any one of (15) to (18) above, wherein the amount of the patient plasma allowed to come into contact with the outer surface of said hollow fiber is between $5 \times 10^{-5}$ and $5 \times 10^{-4}$ m$^3$, the outer surface area of the hollow fiber allowed to come into contact with the plasma is between 0.001 and 1 m$^2$, and a differential pressure caused by aspiration is between 0.001 and 0.08 MPa.

(20) The fibrin-containing biological scaffold according to any one of (1) to (19) above, wherein said fibrinogen activator is thrombin.

(21) The fibrin-containing biological scaffold according to (20) above, wherein said thrombin is obtained from the blood of a single person, from which fibrinogen is obtained.

(22) The fibrin-containing biological scaffold according to any one of (1) to (21) above, which is used in culture of cells selected from the group consisting of vascular endothelial cells, fibroblasts, keratinocytes, mesenchymal stem cells, osteocytes, osteoblasts, osteoclasts, liver cells, pancreatic cells, and hematopoietic stem cells.

(23) The fibrin-containing biological scaffold according to any one of (1) to (22) above, which has a cell growth-stimulating activity that is greater than those in the cases of culturing the cells with no biological scaffold or with a purified fibrinogen concentrate as a control, when at least one type of cells selected from the group consisting of vascular endothelial cells, fibroblasts, keratinocytes, mesenchymal stem cells, osteocytes, osteoblasts, osteoclasts, liver cells, pancreatic cells, and hematopoietic stem cells is cultured.

(24) A method for culturing cells or regenerating tissues, which comprises culturing cells using the fibrin-containing biological scaffold according to any one of (1) to (23) above.

(25) The method according to (24) above, wherein said cells are selected from the group consisting of vascular endothelial cells, fibroblasts, keratinocytes, mesenchymal stem cells, osteocytes, osteoblasts, osteoclasts, liver cells, pancreatic cells, and hematopoietic stem cells.

(26) The method according to (24) or (25) above, wherein the above cells are derived from a single person, from who plasma used as a starting material for a fibrinogen concentrate is collected.

(27) The method according to any one of (24) to (26) above, wherein the culture is carried out in the presence of a substance that stimulates cell growth and/or differentiation.

(28) The method according to (27) above, wherein said substance that stimulates cell growth and/or differentiation is a substance which is released from platelets.

(29) The method according to (28) above, wherein said substance released from platelets is obtained by a method comprising the following steps:

(1) a step of allowing the whole blood to flow through a first-stage filter for giving passage to erythrocytes, platelets and plasma, and adsorbing leukocytes, so as to obtain fractions permeated through the filter;

(2) a step of allowing the permeated fractions obtained in (1) above to flow through a second-stage filter for adsorbing platelets and giving passage to erythrocytes, so as to obtain a filter on which platelets are adsorbed; and

(3) a step of allowing a recovery solution containing a platelet activator to flow through the filter obtained in (2) above, so as to obtain a solution containing an activated platelet-released substance.

(30). The method according to (29) above, wherein said platelet activator is at least one substance selected from the group consisting of ATP, ADP, collagen, and thrombin.

(31) The method according to (27) above, wherein said substance that stimulates cell growth and/or differentiation is a substance which is released from leukocytes.

(32) The method according to (31) above, wherein said substance released from leukocytes is obtained by a method comprising the following steps:

(1) a step of allowing the whole blood to flow through a first-stage filter for giving passage to erythrocytes, platelets and plasma, and adsorbing leukocytes, so as to obtain a filter on which leukocytes are adsorbed; and

(2) a step of allowing a recovery solution containing a leukocyte activator to flow through the filter obtained in (1) above, so as to obtain a solution containing an activated leukocyte-released substance.

(33) The method according to (27) above, wherein said substance that stimulates cell growth and/or differentiation is a mixture consisting of a substance released from platelets and a substance released from leukocytes.

(34) The method according to (33) above, wherein said mixture consisting of a substance released from platelets and a substance released from leukocytes is obtained by a method comprising the following steps:

(1) a step of allowing the whole blood to flow through a filter for giving passage to erythrocytes and plasma and adsorbing platelets and leukocytes, so as to obtain a filter on which platelets and leukocytes are adsorbed; and

(2) a step of allowing a recovery solution containing a platelet activator and a leukocyte activator to flow through the filter obtained in (1) above, so as to obtain a solution containing an activated platelet-released substance and an activated leukocyte-released substance.

(35) The method according to any one of (24) to (34) above, wherein the cells are obtained by allowing cells derived from a human to flow through a filter.

(36) The method according to any one of (24) to (34) above, wherein the plasma used as a starting material for a fibrinogen concentrate is obtained by allowing human blood to flow through a filter.

(37) A cell culture or regenerated tissue supported on a scaffold, which is obtained by the method according to any one of (24) to (36) above.

(38) A method for promoting tissue regeneration, wherein the cell culture or regenerated tissue according to (37) above is applied to damaged tissues, or is used as a graft.

(39) A method for promoting tissue regeneration, which comprises a step of adding to damaged tissues a mixture obtained by mixing the biological scaffold according to any one of (1) to (23) above and cells.

(40) The method for promoting tissue regeneration according to (39) above, wherein said cells are at least one type of cells selected from the group consisting of vascular endothelial cells, fibroblasts, keratinocytes, mesenchymal stem cells, osteocytes, osteoblasts, osteoclasts, liver cells, pancreatic cells, and hematopoietic stem cells.

(41) A concentration system for obtaining a fibrin-containing biological scaffold, which comprises the following means:

(1) a means for roughly purifying human plasma by a plasma component fractionation membrane;

(2) a means for introducing human plasma into the surface of said membrane; and

(3) a means for obtaining a fibrinogen concentrate from the surface of said membrane.

(42) The system according to (41) above, which is characterized in that the cutoff value of said plasma component fractionation membrane is between 80,000 daltons and 300,000 daltons.

(43) The system according to (41) above, which is characterized in that the cutoff value of said plasma component fractionation membrane is between 150,000 daltons and 400,000 daltons.

(44) The concentration system according to (42) or (43) above, wherein said plasma component fractionation membrane is a hollow fiber membrane.

(45) The system according to (44) above, which is characterized in that the material for said hollow fiber membrane is any one selected from the group consisting of hydrophilic polysulfone, EVAL (ethylene-vinyl alcohol copolymer), PAN (polyacrylonitrile), CDA (cellulose diacetate), and CTA (cellulose triacetate).

(46) The system according to (45) above, wherein said material for the hollow fiber membrane is hydrophilic polysulfone or EVAL (ethylene-vinyl alcohol copolymer).

(47) The concentration system according to any one of (41) to (46) above, wherein said introducing means is a liquid-supplying or liquid-aspirating device for introducing human plasma from one of flow ports provided on said fractionation device into the inner or outer membrane surface of the hollow fiber membrane and discharging it from another flow port.

(48) The concentration system according to any one of (41) to (47) above, wherein said means for obtaining said concentrate is a means for storing the concentrate that is connected to one of the flow ports provided on said fractionation device.

(49) The concentration system according to any one of (41) to (48) above, wherein said rough purification means is a plasma component fractionation device where both ends of a hollow fiber membrane built in a vessel are potted such that the inner portion of the hollow is communicated with the outer portion of the vessel.

(50) The concentration system according to any one of (41) to (48) above, wherein said rough purification means is a plasma component fractionation device where one end of a hollow fiber membrane built in a vessel is potted such that the inner portion of the hollow is communicated with the outer portion of the vessel, and the other end is sealed.

(51) A method for operating the concentration system according to (49) above, which comprises supplying human plasma to a hollow portion of the follow fiber in a plasma component fractionation device, allowing mainly liquid components to permeate from the inner surface of the hollow fiber to the outer surface thereof, and collecting a fibrinogen concentrate from the end of the hollow fiber.

(52) The method for operating the concentration system according to (51) above, wherein the ratio (Bin/Bout) between the amount of the patient plasma supplied to said hollow portion per unit time (Bin) and the amount of fibrinogen concentrate collected from the end of the hollow fiber per unit time (Bout) is within the range between 2 and 20.

(53) The method for operating the concentration system according to (52) above, wherein said Bin/Bout ratio is within the range between 5 and 10.

(54) A method for operating the concentration system according to (50) above, which comprises allowing human plasma to come into contact with the outer surface of a hollow fiber in a plasma component fractionation device, allowing mainly liquid components to permeate from the outer surface of the hollow fiber to the inner surface thereof, and concentrating the plasma allowed to be contacted with the outer portion of the hollow fiber, so as to obtain a fibrinogen concentrate.

(55) The method for operating the concentration system according to (54) above, which comprises allowing plasma to come into contact with the outer surface of a hollow fiber, and depressurizing the hollow portion of the hollow fiber when mainly liquid components are allowed to permeate from the outer surface of the hollow fiber to the inner surface thereof, so that said components are allowed to permeate by aspiration from the outer portion of the hollow fiber to the inner portion thereof.

(56) The method for operating the concentration system according to (55) above, wherein said ratio Cinitial/Cend between the amount (Cinitial) of plasma that is allowed to come into contact with the outer surface of said hollow fiber and the amount (Cend) of a fibrinogen concentrate obtained by allowing mainly liquid components to permeate and concentrating the plasma that is allowed to be contacted with the outer surface of the hollow fiber is within the range between 2 and 20.

(57) The method for operating the concentration system according to (54) above, wherein said ratio Cinitial/Cend is within the range between 5 and 10.

(58) The method for operating the concentration system according to any one of (54) to

(57) above, wherein the amount of the patient plasma allowed to come into contact with the outer surface of said hollow fiber is between $5 \times 10^{-5}$ and $5 \times 10^{-4}$ m$^3$, the outer surface area of the hollow fiber allowed to come into contact with the plasma is between 0.001 and 1 m$^2$, and a differential pressure caused by aspiration is between 0.001 and 0.08 MPa.

(59) A system for producing a fibrin-containing biological scaffold, which comprises the following means:

(1) a means for fractionating human plasma by a plasma component fractionation membrane having a cutoff value between 80,000 daltons and 300,000 daltons, so as to separate a fibrinogen concentrate from the residual fractionated plasma;
(2) a means for recovering said fibrinogen concentrate and the residual fractionated plasma, separately;
(3) a means for producing fibrin glue from said fibrinogen concentrate; and
(4) a means for recycling the residual fractionated plasma.

(60) A system for producing a fibrin-containing biological scaffold, which comprises the following means:

(1) a means for fractionating human plasma by a plasma component fractionation membrane having a cutoff value between 150,000 daltons and 400,000 daltons, so as to separate a fibrinogen concentrate from the residual fractionated plasma;
(2) a means for recovering said fibrinogen concentrate and the residual fractionated plasma, separately;
(3) a means for producing fibrin glue from said fibrinogen concentrate; and
(4) a means for recycling the residual fractionated plasma.

(61) The system according to (59) or (60) above, wherein said human plasma is plasma which is collected by continuous extracorporeal circulation.

(62) The system according to any one of (59) to (61) above, wherein said means for collecting human plasma has a means for separating plasma from the whole blood.

(63) The system according to any one of (59) to (62) above, wherein said means for separating human plasma is gravity separation, centrifugation, or a membrane separation means.

(64) The system according to any one of (59) to (63) above, wherein activated thrombin plasma is prepared from human plasma that is used to obtain a fibrinogen concentrate.

(65) The system according to any one of (59) to (64) above, wherein an activated thrombin solution is obtained from said fibrinogen concentrate.

(66) The system according to any one of (59) to (65) above, wherein the residual fractionated plasma obtained as a result of the fractionation by a plasma component fractionation membrane is used to prepare activated thrombin plasma.

(67) The system according to any one of (59) to (66) above, wherein the residual fractionated plasma obtained as a result of the fractionation by a plasma component fractionation membrane is mixed with plasma-separated blood, from which plasma has been separated by a plasma separation means, and the mixture is then returned to a human body.

(68) The system according to any one of (59) to (67) above, wherein said recovery system has a means for storing the obtained fibrinogen concentrate in the form of plasma containing a fibrinogen concentrate derived from a single donor.

(69) The system according to any one of (59) to (68) above, wherein said means for producing fibrin glue has a means for mixing a fibrinogen concentrate, a fibrin stabilizing factor, and a fibrinogen activating factor.

(70) A method for producing a fibrin-containing biological scaffold, which comprises the following steps:

(1) a step of fractionating human plasma by a plasma component fractionation membrane having a cutoff value between 80,000 daltons and 300,000 daltons, so as to separate a fibrinogen concentrate from the residual fractionated plasma;
(2) a step of recovering separately a high molecular weight fractionated plasma containing a large amount of fibrinogen concentrate and the residual fractionated plasma;
(3) a step of producing fibrin glue from said high molecular weight fractionated plasma containing a large amount of fibrinogen concentrate; and
(4) a step of recycling the residual fractionated plasma.

(71) A method for producing a fibrin-containing biological scaffold, which comprises the following steps:

(1) a step of fractionating human plasma by a plasma component fractionation membrane having a cutoff value between 150,000 daltons and 400,000 daltons, so as to separate a fibrinogen concentrate from the residual fractionated plasma;

(2) a step of recovering separately a high molecular weight fractionated plasma containing a large amount of fibrinogen concentrate and the residual fractionated plasma;

(3) a step of producing fibrin glue from said high molecular weight fractionated plasma containing a large amount of fibrinogen concentrate; and

(4) a step of recycling the residual fractionated plasma.

(72) The method according to (70) or (71) above, which comprises a step of collecting said human plasma by continuous extracorporeal circulation.

(73) The method according to any one of (70) to (72) above, wherein said step of collecting human plasma comprises a step of separating plasma from the whole blood.

(74) The method according to any one of (70) to (73) above, wherein said step of separating plasma is gravity separation, centrifugation, or a membrane separation step.

(75) The method according to any one of (70) to (74) above, wherein the residual fractionated plasma obtained as a result of the fractionation by a plasma component fractionation membrane is mixed with plasma-separated blood, from which plasma has been separated by the plasma separation means, and the mixture is then returned to a human body.

(76) The method according to any one of (70) to (75) above, wherein said recovery step comprises a step of storing the obtained fibrinogen concentrate in the form of fibrinogen concentrated plasma derived from a single donor.

(77) The method according to any one of (70) to (76) above, wherein the step of producing fibrin glue comprises a step of mixing a high molecular weight fractionated plasma containing a large amount of fibrinogen, a fibrin stabilizing factor, and a fibrinogen activating factor.

The present invention enables the production of a scaffold with high performance suitable for cell growth and differentiation in regenerative medicine. It also enables the construction of a method for rapidly and simply producing a safe, stable fibrin concentrate having no risk of infection, and a production system thereof.

BRIEF DESCRIPTION OF THE DRAWINGS

Figure 1 is a view showing a fibrinogen concentration system by the Endstop method.

Figure 2 is a view showing a fibrinogen concentration system by the Discard method.

Figure 3 is a view showing a fibrinogen concentration system by the Aspirate method.

Figure 4 is a view showing a fibrinogen concentration system by the Aspirate method, which is incorporated into a blood bag.

Figure 5 is a view showing a fibrinogen concentration system by the Aspirate method, which is incorporated into a blood bag (multistage aspiration).

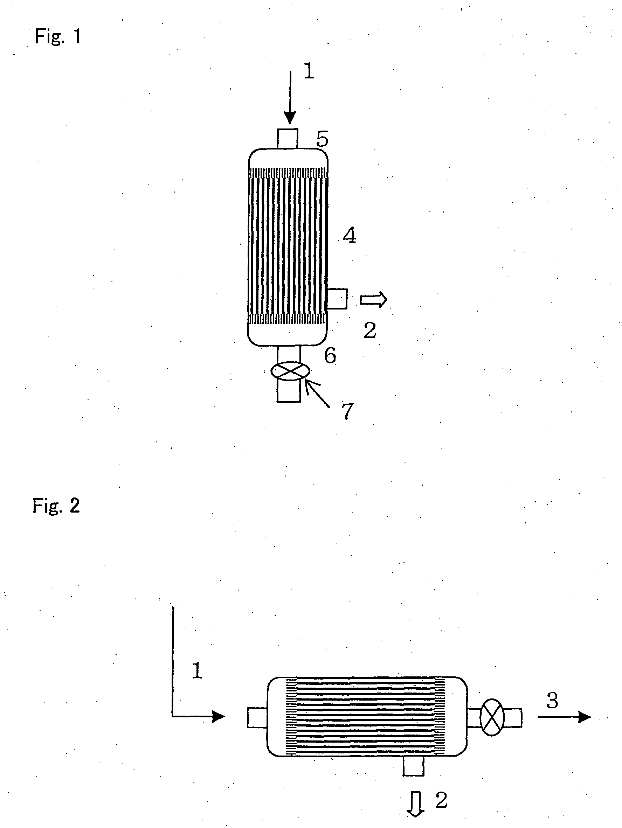

In Figures 1 to 5, 1 represents starting material plasma, 2 represents mainly a liquid component, 3 represents a concentrate, 4 represents a hollow fiber membrane, 5 represents a starting material plasma inlet, 6 represents a concentrate outlet, 7 represents a valve, 8 represents a hollow fiber membrane, the end of which is sealed or is in a loop form, and 9 also represents a hollow fiber membrane, the end of which is sealed or is in a loop form.

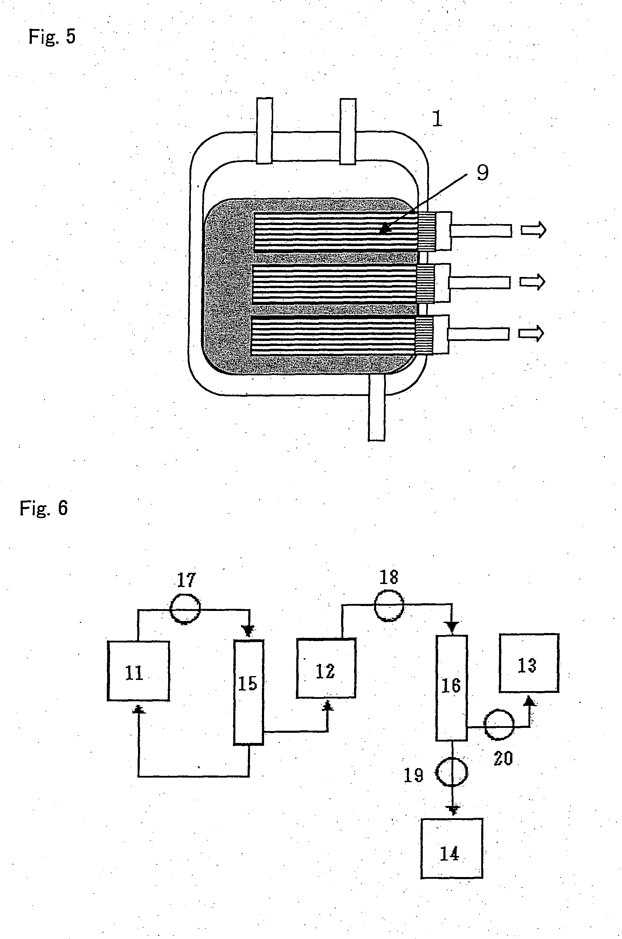

Figure 6 shows an example of a blood treatment device into which the system for producing a biological scaffold of the present invention is incorporated.

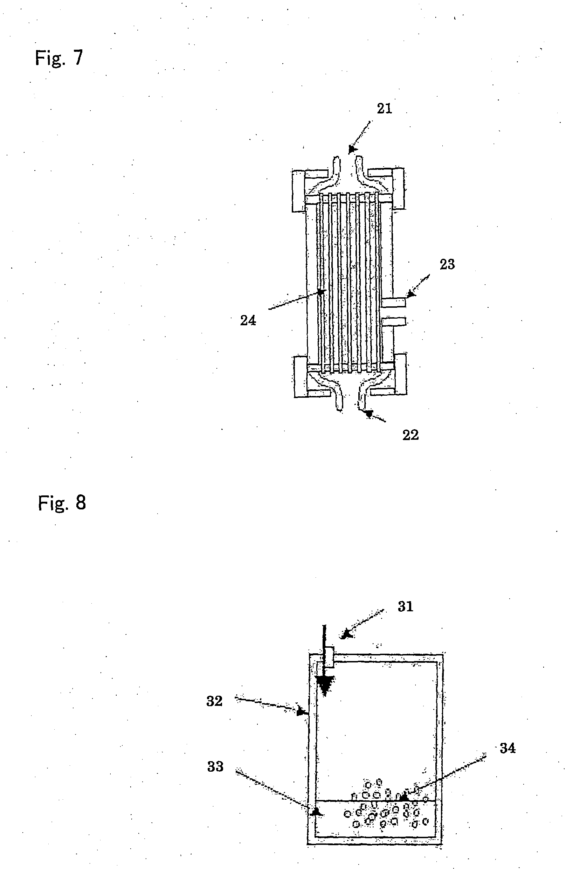

Figure 7 shows an example of a plasma separation column and that of a plasma component fractionation column that are used in the present invention.

Figure 8 shows an example of an activated thrombin plasma preparation device used in the present invention.

In Figures 6 to 8, 11 represents a CPD-added whole blood storage tank, 12 represents a plasma storage tank, 13 represents a filtrated plasma storage tank, 14 represent a concentrated plasma storage tank, 15 represents a plasma separation column, 16 represents a plasma component fractionation column, 17 to 20 represent pumps for blood circuits, 21 represents an inlet for blood or plasma, 22 represents an outlet for a plasma-eliminated blood cell-rich fraction or concentrated plasma, 23 represents an outlet for plasma or filtrated plasma, 24 represents a hollow fiber membrane, 31 represents an inlet for filtrated plasma, 32 represents a plasma storage bag made from vinyl chloride, 33 represents a calcium solution, and 34 represents silicon beads.

BEST MODE FOR CARRYING OUT THE INVENTION

The present invention will be more specifically described below.

The term "biological scaffold" is defined as a material for constituting a support structure (scaffold), which allows cells to anchor, grow and differentiate at a site for tissue regeneration, so as to reconstitute tissues. Other than the aforementioned promotion of the anchoring, growth, and cell differentiation, roles of a biological scaffold may also include securing of a space for regeneration, supply of oxygen and nutrients to cells, and storage of growth factors. An example of a biological scaffold that is practically used for living tissues may be a biopolymer known as an extracellular matrix, such as collagen or laminin. In addition to such biopolymers, the development of biological scaffolds consisting of various types of synthetic polymers or inorganic materials has been studied. According to the recent studies, it has been reported that fibrin has activity as a biological scaffold (Cell Transplantation, 7, 309-317, 1998; and Burn, 24, 621-630, 1998, etc.).

The fibrin-containing biological scaffold of the present invention comprises a mixture of a fibrinogen concentrate obtained from human plasma by short-time rough purification step and a fibrinogen activator.

In one embodiment, the above-described fibrinogen concentrate is obtained by a method comprising steps of cooling plasma for a short time, rapidly thawing the plasma, and recovering the fibrinogen concentrate (for example, a centrifugation step).

Specifically, the above-described fibrinogen concentrate can be obtained by a method comprising steps of cooling the plasma for 10 to 60 minutes, thawing the plasma for 15 to 60 minutes, and recovering the fibrinogen concentrate. Moreover, the cooling step is preferably carried out at a temperature between -20°C and -40°C, and the thawing step is preferably carried out at a temperature between -10°C and +15°C. In terms of biocompatibility, human plasma is preferably used as plasma which is a starting material, when a human is targeted. When an animal is targeted, the plasma of the same animal is preferably used.

More specific embodiments of a method for obtaining the above-described fibrinogen concentrate are as follows. First, the plasma obtained by centrifugation (4°C) of the whole blood is left at rest at a low temperature of approximately -30°C for 30 minutes, so as to freeze it. Thereafter, it is left at -2.5°C for 30 minutes, and then at 12°C for 30 minutes. Thereafter, it is left at 4°C for 30 minutes. Finally, it is centrifuged (1,000 g x 15 minutes, 1°C) to eliminate the supernatant, thereby obtaining a fibrinogen concentrate. Otherwise, there is another method comprising leaving at rest the plasma obtained by centrifugation of the whole blood (1,000 g x 15 minutes, 4°C) at a low temperature of approximately -30°C for 60 minutes to freeze it, leaving it at -5°C for 30 minutes and then 4°C for 30 minutes, and finally centrifuging it (1,000 g x 15 minutes, 1°C) to eliminate the supernatant, thereby obtaining a fibrinogen concentrate.

It is to be noted that a fibrinogen concentrate can be obtained not only by centrifugation, but also by decantation, filtration, or other operations. Accordingly, the operation used in the present invention is not limited to centrifugation.

In this method, a fibrinogen concentrate is obtained from plasma for a short period of time of 1 to 2 hours (refer to National Publication of International Patent Application No. 2001-513073). This method is referred to as an "improved cryo method" in the present specification.

The thus obtained fibrin-containing biological scaffold of the present invention contains a fibrinogen concentrate that is obtained from the plasma by precipitation, resulting in a recovery rate of fibrinogen within the range between 15% and 32%.

As described later in the examples, the present inventors have found that in the aforementioned method involving freezing and thawing steps (improved cryo method), the activity of a biological scaffold becomes high, when the recovery rate is adjusted to be in the range between 15% and 32%.

In the present specification, the term "fibrinogen recovery rate" is used to mean a value obtained by the following formula:

$$\text{Fibrinogen recovery rate} = (\text{the amount of fibrinogen contained in a fibrinogen}$$

$$\text{concentrate/ the amount of fibrinogen contained in plasma as a starting material used to}$$

$$\text{prepare the fibrinogen concentrate}) \times 100$$

A quantification of fibrinogen can be carried out by any method such as the thrombin time method which comprises adding thrombin and $Ca^{2+}$ to a sample and measuring a time necessary for generating fibrin clots; the weighing method of weighing the generated fibrin clots; or the immunoassay using anti-fibrinogen antibody. In terms of the simplicity of operations, the thrombin time method is preferable.

In another aspect of the present invention, a fibrinogen concentrate can also preferably be obtained for a short period of time of 1 to 2 hours by concentration operations using a plasma component fractionation device comprising therein a hollow fiber membrane. The hollow fiber used in concentration preferably has a cutoff value at which fibrinogen is not permeable but mainly liquid components are permeable. "Cutoff value" is defined as a molecular weight of a solute (generally, polyethylene glycol is used) with a rejection rate of 90%. In this case, the term "mainly liquid components" is used to mean liquid components coexisting with substances having a size smaller than that of fibrinogen. Among such substances having a size smaller than that of fibrinogen, albumin may be either permeable or not permeable, depending on purposes or conditions. By selecting an appropriate hollow fiber, albumin is allowed to permeate as a component of a permeable solution, or it is not allowed to permeate but remains in a concentrate.

The cutoff size is preferably between 30,000 and 1,500,000 daltons, and more preferably between 50,000 and 900,000 daltons. If the cutoff size is in this range, fibrinogen with a size of about 350,000 daltons can efficiently be concentrated, and a high throughput can be achievable under suitable operation conditions. Within the above-described range, a hollow fiber membrane with a more preferred cutoff size can be selected depending on purposes. If a molecular weight cut off between 80,000 and 300,000 is selected, it is also possible to simultaneously concentrate a growth factor or the like having a relatively low molecular weight. Thus, a hollow fiber membrane is preferably selected depending on purposes. If a molecular weight cut off between 150,000 and 400,000 is selected, although the degree of concentration of a component having a relatively low molecular weight, such as a growth factor, is not high, a matrix protein with a relatively high molecular weight is concentrated together with fibrinogen, and an increase in pressure during the concentration operation can be maintained at sufficiently low. In particular, it is effective for reduction of the time necessary for such a concentration operation. When a membrane having a molecular weight cut off of more than 350,000 daltons is used, the leakage of fibrinogen is likely to occur to a certain extent. In this case also, if the molecular weight cut off is 1,500,000 daltons or less, a certain degree of concentration can be obtained. Even when a degree of concentration is relatively low, for example, even when such concentration rate is 3 to 5 times, sufficient cell growth ability can be obtained depending on conditions.

Concentration of fibrinogen with a hollow fiber differs from the freezing and thawing methods (the cryo method, the improved cryo method, etc.). Components useful for a biological scaffold are likely to be concentrated together with the fibrinogen. Thus, although the recovery rate of fibrinogen is high, the activity as a biological scaffold is often high. When the recovery rate of fibrinogen obtained by the hollow fiber method is low, it is approximately 20%. However, when it is high, a high recovery rate of almost 100% can be realized.

The material for a hollow fiber can be selected as appropriate, depending on purposes or conditions. Examples of such a material may include: polyolefin resins such as polyethylene, an ethylene/vinyl alcohol copolymer, polypropylene, or poly-4-methyl-2-pentene; fluorocarbon resins such as a polyvinylidene fluoride resin, an ethylene/tetrafluoroethylene resin, or a polychlorotrifluoroethylene resin; various types of synthetic polymers such as polysulfone, polystyrene, an acrylic resin, nylon, polyester, polycarbonate, polyacrylamide, or polyurethane; natural polymer such as agarose, cellulose, cellulose acetate, chitin, chitosan, or alginate; inorganic materials such as hydroxyapatites, glass, alumina, or titania; and metals such as stainless, titanium, or aluminum.

In particular, when a hydrophobic material is adopted, a hollow fiber having an pour surface that is appropriately modified can be selected. For example, a hollow fiber whose surface is modified to be hydrophilic can efficiently avoid clogging in many cases, and thus, it is preferably selected. A method of modifying the surface basically involves the construction of a hydrophilic surface. Known methods can be adopted depending on purposes. For example, ionizing radiation is applied to the surface, and the surface is then treated with hot water, so as to modify the surface to be hydrophilic. Otherwise, the modification of the surface can also be carried out by coating the surface with an amphipathic polymer. Examples of such a polymer may include: hydroxyl acrylic or methacrylic polymers such as hydroxyethyl acrylate, hydroxyethyl methacrylate, hydroxypropyl acrylate, or hydroxypropyl methacylate; amine polymers; polyethylene glycol polymers; and copolymers thereof. Of these, a hydroxyethyl methacrylate-dimethylaminoethyl methacrylate copolymer has been commonly used as a coating agent for blood treatment, and thus, it is preferably adopted herein (Japanese Patent Application Laid-Open No. 10-234361). In addition, the graft polymerization method is a method of allowing a hydrophilic polymer to chemically bind to a filter carrier. This method is advantageous in that it causes no worry about elution. The method described in Japanese Patent Application Laid-Open No. 2000-185094 and the like is preferably adopted.

Among the aforementioned materials, materials used in blood treatment, for example, those that have been commonly used as dialysis membranes or membranes for extracorporeal circulation, are preferably adopted. Examples of a material that is preferably used herein may include hydrophilized polysulfone, hydrophilized polyethersulfone, a polyethersulfone-polyarylate resin polymer alloy, polyallylethersulfone, an ethylene/vinyl alcohol copolymer, polyacrylonitrile, cellulose diacetate, cellulose triacetate, hydrophilized polypropylene, and a polyester coated with a hydrophilic polymer.

A fibrinogen concentration system into which the aforementioned hollow fiber is incorporated will be described. The present system is a concentration system for obtaining a fibrin-containing biological scaffold, which comprises the following means:

(1) a means for roughly purifying human plasma by a plasma component fractionation membrane;

(2) a means for introducing human plasma into the surface of above-described membrane; and

(3) a means for obtaining a fibrinogen concentrate from the surface of above-described membrane.

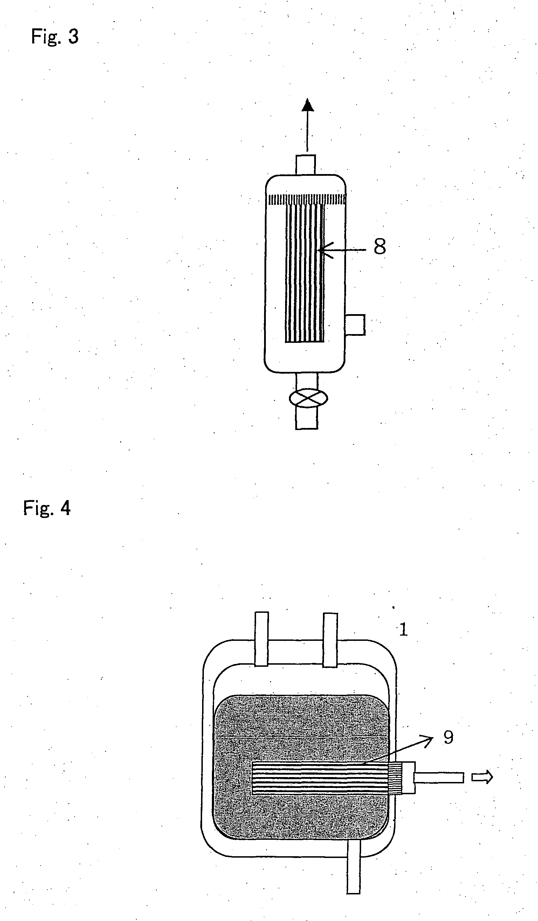

The summary of the present system is shown in Figures 1 to 5. Each of Figures 1 and 2 shows a plasma component fractionation device, wherein both ends of a hollow fiber membrane built in a vessel are potted, such that the inner portion of the hollow is communicated with the outer portion of the vessel. Each of Figures 3 to 5 shows another plasma component fractionation device, wherein one end of a hollow fiber membrane built in a vessel is potted such that the inner portion of the hollow is communicated with the outer portion of the vessel, and the other end is sealed. In addition, as a system corresponding to the system shown in each of Figures 3 to 5, it is also possible to construct another plasma component fractionation device, wherein both ends of a hollow fiber membrane built in a vessel are potted such that the inner portion of the hollow fiber is communicated with the outer portion of the vessel and also such that the hollow fiber is bent in a U form so that the both ends of the hollow fiber membrane is unified in the same direction. These systems involve a liquid-supplying or liquid-aspirating device for introducing human plasma from one of flow ports provided on the above-described fractionation device into the inner or outer membrane surface of the hollow fiber membrane and discharging it from another flow port. A fibrinogen concentrate can preferably be collected in a concentrate storage means that is connected to one of flow ports provided on the above-described fractionation device. More specific descriptions will be given below.

When permeation is carried out from the hollow portion (internal side) of a hollow fiber to the outer portion (external side) thereof, the following methods can be applied: a method comprising sealing the end of a hollow portion, treating plasma, and recovering a concentrate contained in the hollow portion of the hollow fiber by a recovery solution (Figure 1, the Endstop method); a method comprising opening the end of a hollow portion and continuously collecting a concentrate from the end of the hollow fiber (Figure 2, the Discard method); and a method for allowing mainly liquid components to permeate from the outer portion to the hollow portion (Figure 3, the Aspirate method). In the case of the Endstop method, since the volume of a hollow portion is small, there is only a small space for storing fibrinogen of interest. Thus, an increase in the pressure is likely to occur due to clogging during plasma treatment. Accordingly, the Discard method is applied more preferably than the Endstop method. In the case of the Discard method, the ratio (Bin/Bout) between the amount (Bin) of patient plasma supplied to a hollow portion of hollow fiber per unit time (Bin) and the amount (Bout) of fibrinogen concentrate collected from the end of the hollow fiber per unit time is preferably between 2 and 20, and more preferably between 5 and 10. When the Bin/Bout ratio is within this range, it is possible to set conditions where a fibrinogen concentrate is efficiently obtained while avoiding an increase in the pressure.

The Aspirate method is used in the case of allowing mainly liquid components to permeate from the outer portion to the hollow portion. There may be two cases: a case of allowing mainly liquid components to permeate by pressurizing plasma from the outer portion; and a case of allowing mainly liquid components to permeate by depressurizing the inside of the hollow portion thereby aspirating them. In the case of aspiration, the pressure is not applied to the side of the concentrate. Thus, it is possible to prevent degeneration of the concentrate of interest. Accordingly, it becomes possible to set a pressure higher than that in the case of pressurization. In principle, a differential pressure generated as a result of aspiration cannot be set at more than the atmospheric pressure. However, by applying a differential pressure that is close to the atmospheric pressure, such as a differential pressure of 0.05 MPa or higher, efficient concentration operations can be realized. As in the case of the Discard method, the ratio (Cinitial/Cend) between the amount (Cinitial) of plasma that is allowed to come into contact with the outer surface of a hollow fiber and the amount (Cend) of the obtained fibrinogen concentrate is preferably between 2 and 20, and more preferably between 5 and 10. When the ratio Cinitial/Cend is within this range, it is possible to set conditions where a fibrinogen concentrate is efficiently obtained while avoiding an increase in the pressure. Moreover, preferred conditions for the Aspirate method preferably consist of the amount of plasma being between $5 \times 10^{-5}$ and $5 \times 10^{-4}$ m$^3$, the outer surface area of a hollow fiber allowed to come into contact with the plasma being between 0.001 and 1 m$^2$, and a differential pressure caused by aspiration being between 0.001 and 0.08 MPa. More preferably, such conditions consist of the amount of plasma being between $1 \times 10^{-4}$ and $4 \times 10^{-4}$ m$^3$, the outer surface area of a hollow fiber allowed to come into contact with the plasma being between 0.01 and 0.5 m$^2$, and a differential pressure caused by aspiration being between 0.01 and 0.08 MPa.

Next, especially the aspirate method will be described in detail, using figures. Figure 3 shows a configuration wherein a hollow fiber is introduced into a common module case. In contrast, Figure 4 shows a configuration wherein a hollow fiber is introduced into a blood bag. By introducing plasma into such a blood bag, it becomes possible to directly obtain a fibrinogen concentrate from the plasma by the aspiration method. Figure 5 shows a configuration wherein plural number of hollow fiber bundles are introduced into a blood bag by the Aspirate method, so as to carry out the aspiration operation at multi-stages. As shown in the figure, the plural number of hollow fiber bundles are provided in a vertical direction, and the aspiration operation is carried out from the upper side. If an increase in pressure occurs due to clogging, then the routine proceeds to aspiration with hollow fibers at the next stage. An appropriate index of such an increase in pressure is between 0.05 MPa and 0.07 MPa.

In all of the Endstop method, the Discard method, and the Aspirate method, after a concentrate having a certain concentration has been obtained, the obtained concentrate can be concentrated by another operation. Examples of such an operation that is conducted after a concentrate has been obtained may include centrifugation, and precipitation by freezing and thawing. The combined use of concentration by hollow fibers and other concentration methods as described above enables the efficient obtainment of a concentrate with a high concentration rate.

The fibrin-containing biological scaffold of the present invention further comprises a fibrinogen activator. The term "fibrinogen activator" is used to mean a substance having an action to convert fibrinogen into fibrin. An example thereof is thrombin.

As such thrombin, those having biological activity as thrombin, for example, those obtained by fractionation of a plasma protein, can be used. For example, a product prepared by purifying prothrombin from human or bovine plasma, and preferably from human plasma that is also used in preparation of a fibrinogen concentrate, and allowing thromboplastin to act on the prothrombin in the presence of $Ca^{2+}$, can be used. Otherwise, products that are commercially available from pharmacies may be used.

The amount of thrombin mixed in a scaffold may be set between 0.01 and 100 units, and preferably between 0.1 and 10 units, with respect to 1 mg of a fibrinogen concentrate.

The fibrin-containing biological scaffold of the present invention comprises a substance derived from plasma, which is associated with the formation and stabilization of fibrin. Examples of such a substance derived from plasma which is associated with the formation and stabilization of fibrin may include factor XIII and fibronectin.

The fibrin-containing biological scaffold of the present invention can preferably be used in the culture of cells used in the regenerative medicine of the skin, cartilage, bone, liver, kidney, cornea, etc. Examples of such cells may include human-derived stem cells (in particular, bone marrow derived mesenchymal stem cells), endothelial cells, epithelial cells, parenchymal cells (in particular, hepatic parenchymal cells), fibroblasts, keratinocytes, osteocytes, osteoblasts, osteoclasts, and hematopoietic stem cells.

A method of culturing the above-described cells using the fibrin-containing biological scaffold of the present invention will be described. A method of culturing the cells is not particularly limited. A method of adding a mixture of cells and a medium to a scaffold and then leaving the obtained mixture in a certain atmosphere at a certain temperature, or the like can be used.

Medium used herein is not particularly limited, and either a serum containing medium or a serum-free medium can be used. Examples of such a serum containing medium may include MEM medium, BME medium, DME medium αMEM medium, IMEM medium, ES medium, DM-160 medium, Fisher's medium, F12 medium, WE medium, RPMI medium, and a medium formed by adding serum to a mixture of basal mediums aforementioned.

An atmosphere used in cell culture is not particularly limited. For example, an atmosphere such as a mixture of carbon dioxide and the air can be used. The culture temperature applied in cell culture is preferably between 10°C and 50°C°, and particularly preferably between 30C° and 40°C, at which the cell growth and differentiation actively take place. Examples of a method of recovering the culture product obtained according to the aforementioned method by removing it from the scaffold may include: a method of decreasing an ambient temperature or the temperature of the scaffold; a method of exchanging the medium with a low-temperature medium; a method using addition of a chelating agent such as EDTA or a method using enzyme treatment such as trypsin or collagenase; and a method of mechanically removing the culture product using a cell scraper.

The aforementioned cell culture may also be carried out in the presence of a substance that stimulates cell growth and differentiation. Preferred examples of such a substance that stimulates cell growth and differentiation may include a substance released from platelets, a substance released from leukocytes, and a mixture thereof, which are obtained by the methods described later. Otherwise, the following growth factors, which are generally used as substances which stimulate cell growth and differentiation in the regenerative medicine field, may also be used: a fibroblast growth factor, a transforming growth factor, an insulin-like growth factor, a hepatocyte growth factor, a vascular endothelial growth factor, a heparin-binding endothelial cell growth factor, and a connective tissue growth factor.

Specific examples of the aforementioned substance released from platelets may include ATP, ADP, collagen, and thrombin.

Such a substance released from platelets is obtained by a method comprising the following steps:

(1) a step of allowing the whole blood to flow through a first-stage filter for giving passage to erythrocytes, platelets and plasma, and adsorbing leukocytes, so as to obtain fractions permeated through the filter;
(2) a step of allowing the permeated fractions obtained in (1) above to flow through a second-stage filter for adsorbing platelets and giving passage to erythrocytes, so as to obtain a filter on which platelets are adsorbed; and
(3) a step of allowing a recovery solution containing a platelet activator to flow through the filter obtained in (2) above, so as to obtain a solution containing an activated platelet-released substance.

The filter described in National Publication of International Patent Application No. 11-508813 is an example of the first-stage filter used in the step described in (1) above. The filter described in Japanese Patent Publication No. 2-13587 is an example of the second-stage filter used in the step described in (2) above.

The term "platelet activator" is used herein to mean ATP, ADP, epinephrine, collagen, thrombin, trypsin, latex particles, and the like. The term "recovery solution containing a platelet activator" is used to mean a solution obtained by dissolving such a platelet activator in a physiological saline, a suitable buffer solution such as a phosphate buffer solution, an albumin solution, or a mixed solution thereof. Such a recovery solution containing a platelet activator can be obtained by allowing platelet to come into contact with the aforementioned platelet activator.

On the other hand, the aforementioned substance released from leukocytes can be obtained by a method comprising the following steps:

(1) a step of allowing the whole blood to flow through a first-stage filter for giving passage to erythrocytes, platelets and plasma, and adsorbing leukocytes, so as to obtain a filter on which leukocytes are adsorbed; and
(2) a step of allowing a recovery solution containing a leukocyte activator to flow through the filter obtained in (1) above, so as to obtain a solution containing an activated leukocyte-released substance.

Examples of a leukocyte activator used herein may include: a substance that is contained in leukocytes themselves and has an activity of stimulating the ability of leukocytes regarding growth, differentiation, adhesion, and migration (e.g., an fMLP peptide, and cytokine such as TNF, IL-1, or IL-6); and an anti-T cell antibody. A recovery solution containing a leukocyte activator can be obtained by allowing a leukocyte activator to come into contact with a surfactant, a suitable buffer solution, or a hypotonic solution, or by mechanical destruction of leukocytes.

A mixture of a substance released from platelets and a substance released from leukocytes is obtained by a method comprising the following steps:

(1) a step of allowing the whole blood to flow through a first-stage filter for giving passage to erythrocytes and plasma and adsorbing platelets and leukocytes, so as to obtain a filter on which platelets and leukocytes are adsorbed; and
(2) a step of allowing a recovery solution containing a platelet activator and a leukocyte activator to flow through the filter obtained in (1) above, so as to obtain a solution containing an activated platelet-released substance and an activated leukocyte-released substance.

The filter described in Japanese Patent Publication No. 2-13587 is an example of the first-stage filter used in the step described in (1) above.

Examples of filters that are preferably used in each of the above-described operations may include a non-woven filter (a plate laminated type or cylindrical laminated type), a hollow-fiber membrane filter, and a porous membrane filter. Known separation filters such as a filter for eliminating leukocytes and giving passage to platelets (WO01/32236) or a filter for eliminating leukocytes and platelets (Japanese Patent Publication No. 02-13587) may be used.

The thus obtained substance released from platelets and substance released from leukocytes can also be used as agents for stimulating cell differentiation or growth even in regenerative medicine wherein fibrin glue is not used as a scaffold.

As described later in the examples, the scaffold of the present invention exhibits excellent activities regarding adhesion, chemotaxis, and promotion of cell growth in fibroblasts. In addition, it also exhibits excellent activity of promoting cell growth in vascular endothelial cells. Moreover, as described later in the examples, substances released from the activated platelets and leukocytes that are obtained by the method of the present invention have cell growth-promoting activity to fibroblasts and vascular endothelial cells. That is to say, using the scaffold of the present invention in the treatment of diabetes, arteriosclerosis obliterans (ASO), and skin injury such as intractable ulcer, that is, using the present scaffold in wound healing, it becomes possible to impart the effects of promoting healing to healing processes that are known as an inflammatory phase and a growth phase (granulation phase), for which the migration and growth of fibroblasts and vascular endothelial cells are required at injured sites. Accordingly, the scaffold of the present invention is also effective for treatment of injuries.

The present invention also provides a system for producing a fibrin-containing biological scaffold. This production system comprises the following means:

(1) a means for fractionating human plasma by a plasma component fractionation membrane having a cutoff value between 80,000 daltons and 900,000 daltons, so as to separate a fibrinogen concentrate from the residual fractionated plasma;
(2) a means for recovering the above-described fibrinogen concentrate and the residual fractionated plasma, separately;

(3) a means for producing fibrin glue from the above-described fibrinogen concentrate; and
(4) a means for recycling the residual fractionated plasma.

The system for producing a biological scaffold of the present invention can be incorporated into, for example, a blood treatment device as shown in Figure 5. The blood treatment device involves a system wherein the double filtration method is applied. The method comprises allowing the blood collected from a living body to pass through a first filtration column (15) for separating plasma, separating components from the plasma filtrated through the first filtration column using a second filtration column (16), and returning the residual plasma to the living body.

As shown in Figure 5, in the conventional blood treatment device, high molecular weight fractions such as LDL or VLDL are eliminated by the second filtration column for fractionating plasma components, and thus, the plasma viscosity is decreased, so as to prevent or treat vascular occlusion due to arteriosclerosis or hyperlipidemia. Thus, high molecular weight fractions separated through a filter have been discarded.

In one embodiment of the present invention, high molecular weight fractions that have conventionally been discarded are utilized to obtain fractions containing a large amount of fibrinogen that is suitable as a material of a fibrin-containing biological scaffold. Thus, the present invention is characterized in that a filter having a specific cutoff value is used for the second filtration column for fractionating plasma components.

As a filter for fractionating plasma components, a hollow fiber membrane that has already been explained in the fibrinogen concentration system may be used.

In the present invention, collection of the blood is carried out using an injection needle or catheter which is connected with the blood vessel of a living body.

As a plasma separation device for collecting plasma from the whole blood, any one of a membrane separation-type device, a centrifugation-type device, and the previously known devices can be used.

A membrane separation-type plasma separation device means a device comprising a housing filled with a hollow fiber-type separation membrane having a pore size that does not allow at least blood cells to filtrate. When the blood is passed through the inside of a hollow fiber, plasma components are separated through the membrane wall of the hollow fiber. The material of a separation membrane is not particularly limited. Examples of a material may include polysulfone, polyethersulfone, polyethylene, polypropylene, cellulose, cellulose acetate, ethylene vinyl alcohol, polyacrylonitrile, Teflon, and polyester. Such a membrane separation-type plasma separation device is particularly preferable in that plasma can be continuously separated from the blood at a constant rate.

A centrifugation-type plasma separation device means a device for introducing the blood into a centrifugal bowl and rotating the bowl, so as to separate plasma using a difference in specific gravity between blood cells and plasma. At the time when a certain degree of separation has been achieved, the blood contained in the bowl is returned to a patient, and a fresh blood is then introduced into the bowl for centrifugation.

In the present invention, the thus obtained plasma fraction containing a large amount of fibrinogen can be directly used as a raw material for producing a fibrin-containing biological scaffold.

Such a fibrin-containing biological scaffold can be obtained by mixing a fibrin-stabilizing factor such as a factor XIII and a fibrinogen-activating factor such as thrombin-calcium into the plasma fraction containing a large amount of fibrinogen obtained in the present invention.

The residual fraction obtained by separating the plasma fraction containing a large amount of fibrinogen through a filter can be used to prepare plasma containing a large amount of thrombin by any methods of activating prothrombin to generate thrombin.

For example, calcium having an optimal concentration is added to the residual fraction, and the mixture is then allowed to come into contact with negative surface charge such as silicon beads, so as to generate thrombin.

Moreover, the residual fraction obtained by separating the plasma fraction containing a large amount of fibrinogen through the second filter is returned to a blood donor, directly or after mixing with a fraction containing large quantities of blood cells separated from plasma by a plasma separation device.

In the present invention, the plasma fraction containing a large amount of fibrinogen separated though the second filter can also be stored.

For example, generally discarded plasma fractions containing a large amount o fibrinogen are stored or conserved in a frozen state or at a low temperature, and then are used as materials for producing fibrin-containing biological scaffolds, in what is called Reopheresis, in which high molecular weight proteins in blood, such as fibrinogen, are eliminated by the extracorporeal circulation method to decrease the viscosity of the blood, so as to treat low vision or toe gangrene caused by circulatory disorder of the blood that is developed in peripheral blood vessels due to abnormally high blood viscosity. Thus, using generally discarded plasma of a patient, what is called autologous fibrin glue can be produced from the blood of the patient. In addition, by gathering these materials, a savings bank for storing fibrin-containing biological scaffolds can also be established.

EXAMPLES

The present invention will be more specifically described in the following examples. However, these examples are not intended to limit the scope of the present invention.

Example 1: Preparation of fibrinogen concentrate by improved cryo method and production of fibrin-containing biological scaffold

400 ml of fresh blood collected from a healthy subject [to which 56 ml of CPD (Citrate-phosphate-dextrose C7165 manufactured by Sigma-Aldrich Inc.) had been added as an anticoagulant] was dispersed into aliquotes into a 50-ml centrifuge tube (No. 352070 manufactured by Nippon Becton Dickinson Co. Ltd.), followed by centrifugation (1,000 g x 15 minutes, 4°C) (No.3740 manufactured by KUBOTA Corp.), so as to obtain 235 ml of plasma. The obtained plasma was then transferred into a freezer (EEV-204N manufactured by Whirlpool Corp.), and it was left at rest at -27°C for 30 minutes, so as to freeze it. Thereafter, the centrifuge tube containing frozen plasma was transferred into a refrigerated centrifuge having a chamber temperature of -2.5°C (No.3740 manufactured by KUBOTA Corp.), and it was then left in chamber for 30 minutes. Subsequently, the centrifuge tube was transferred into another refrigerated centrifuge whose chamber temperature had previously been set at 12°C, and it was left for 30 minutes. Thereafter, the tube was left at rest in a refrigerator (Medicool MPR-510 manufactured by SANYO Electric Co. Ltd, 4°C) for 30 minutes. Finally, centrifugation (1,000 g x 15 minutes, 1°C) was carried out, and the supernatant was removed, so as to obtain a fibrinogen concentrate. Fibrinogen was quantified using a commercially available fibrinogen measurement kit (Pacific Hemostasis kit, Fisher Scientific International) that was based on the thrombin time method. Measurement was carried out in accordance with the procedure manual provided from the manufacturer. The amount of fibrinogen recovered was 40.2 mg. The amount of fibrinogen contained in plasma as a starting material was measured to be 198 mg. Thus, the recovery rate was 20.3% (40.2/198 $\times$ 100 = 20.3). The fibrinogen concentrate was rapidly mixed with a human thrombin solution (T8885 manufactured by Sigma-Aldrich Inc.) (5.0 NIH units/ml) containing 50 mM calcium chloride (C5080 manufactured by Sigma-Aldrich Inc.) with equal volume, so as to produce a fibrin-containing biological scaffold.

Example 2: Preparation of fibrinogen concentrate by improved cryo method (another method) and production of fibrin-containing biological scaffold

40 ml of fresh blood collected from a healthy subject [to which 5.6 ml of CPD (Citrate-phosphate-dextrose C7165 manufactured by Sigma-Aldrich Inc.) had been added as an anticoagulant] was placed in a 50-ml centrifuge tube (No. 352070 manufactured by Nippon Becton Dickinson Co. Ltd.), followed by centrifugation (1,000 g x 15 minutes, 4°C) (No.3740 manufactured by KUBOTA Corp.), so as to obtain 20.5 ml of plasma. The obtained plasma was then transferred into a freezer (EEV-204N manufactured by Whirlpool Corp.), and it was left at rest at -30°C for 60 minutes, so as to freeze it. Thereafter, the centrifuge tube containing frozen plasma was left at rest in a refrigerated centrifuge having a chamber temperature of -5°C (No. 3740 manufactured by KUBOTA Corp.) for 30 minutes. Subsequently, it was left at rest in a refrigerator (Medicool MPR-510 manufactured by SANYO Electric Co. Ltd, 4°C) for 30 minutes. Finally, centrifugation (1,000 g x 15 minutes, 1°C) was carried out, and the supernatant was removed, so as to obtain a fibrinogen concentrate. The amount of fibrinogen recovered was 7.1 mg. The amount of fibrinogen contained in plasma as a starting material was measured to be 24.8 mg. Thus, the recovery rate was 28.6% (7.1/24.8 $\times$ 100 = 28.6). The fibrinogen concentrate was rapidly mixed with a human thrombin solution (T8885 manufactured by Sigma-Aldrich Inc.) (5.0 NIH units/ml) containing 50 mM calcium chloride (C5080 manufactured by Sigma-Aldrich Inc.) with equal volume, so as to produce a fibrin-containing biological scaffold.

Comparative example 1: Preparation of fibrinogen concentrate by cryo method and production of fibrin-containing biological scaffold

Preparation of a fibrinogen concentrate by the cryo method was carried out according to the method described by Casali et al (Transmission, 32, 641-643, 1992). 40 ml of fresh blood collected from a healthy subject [to which 5.6 ml of CPD (Citrate-phosphate-dextrose C7165 manufactured by Sigma-Aldrich Inc.) had been added as an anticoagulant] was placed in a 50-ml centrifuge tube (No. 352070 manufactured by Nippon Becton Dickinson Co. Ltd.), followed by centrifugation (1,000 g x 15 minutes, 4°C) (No. 3740 manufactured by KUBOTA Corp.), so as to obtain 22.5 ml of plasma. The obtained plasma was then transferred into an ultra-low temperature freezer (MDF-293 manufactured by SANYO Electric Co. Ltd.), and it was left at -80°C for 18 hours, so as to freeze it. Thereafter, the frozen plasma was transferred into a refrigerator (MPR-311 manufactured by SANYO Electric Co. Ltd.) that had been set at 4°C, and it was then left for 16 hours, so as to thaw it at a slow speed. Subsequently, it was centrifuged at 1,000 g x 15 minutes with a refrigerated centrifuge (4°C). Freezing and thawing were repeated under the aforementioned conditions, while care is taken not to disturb the formed precipitate. After thawing, centrifugation was carried out again at 4°C at 1,000 g x 15 minutes. The precipitate was recovered, and it was defined as a fibrinogen concentrate. The amount of fibrinogen recovered was 8.7 mg. The amount of fibrinogen contained in the original plasma was measured to be 21.5 mg. Thus, the recovery rate was 40.5% (8.7/21.5 × 100 = 40.5). The fibrinogen concentrate was rapidly mixed with a human thrombin solution (T8885 manufactured by Sigma-Aldrich Inc.) (5.0 NIH units/ml) containing 50 mM calcium chloride (C5080 manufactured by Sigma-Aldrich Inc.) with equal amount, so as to produce a fibrin-containing biological scaffold.

Comparative example 2: Preparation of fibrinogen concentrate by ethanol precipitation method and production of fibrin-containing biological scaffold

Preparation of a fibrinogen concentrate by the ethanol precipitation method was carried out according to the method described by Kjaergard (Surg Gynecol Obstet, 175(1), July, 1992). 40 ml of fresh blood collected from a healthy subject [to which 5.6 ml of CPD had been added as an anticoagulant] was placed in a 50-ml centrifuge tube (No. 352070 manufactured by Nippon Becton Dickinson Co. Ltd.), followed by centrifugation at 600 g x 10 minutes (No. 3740 manufactured by KUBOTA Corp.), so as to obtain plasma. The obtained plasma was then transferred into a new 50-ml centrifuge tube. 2.5 ml of ethanol (99.5%, manufactured by Wako Pure Chemical Industries, Ltd.) was added thereto over 30 minutes on ice water (0°C), while stirring sometimes, so as to precipitate fibrinogen. The fibrinogen precipitate was recovered by centrifugation at 600g x 15 minutes, and it was defined as a fibrinogen concentrate. The amount of fibrinogen recovered was 2.7 mg. The amount of fibrinogen contained in the original plasma was measured to be 21.5 mg. Thus, the recovery rate was 12.7% (2.7/21.5 × 100 = 12.7). The fibrinogen concentrate was stored at -85°C until it was used. Before use, the fibrinogen concentrate was thawed at 37°C. The obtained fibrinogen concentrate was rapidly mixed with 0.3 volume of a human thrombin solution (T8885 manufactured by Sigma-Aldrich Inc.) (5.0 NIH units/ml) containing 80 mM calcium chloride (C5080 manufactured by Sigma-Aldrich Inc.), so as to produce a fibrin-containing biological scaffold.

Test example 1: Cell growth-stimulating activity to normal human fetal lung-derived fibroblasts

0.5 mg of the fibrin-containing biological scaffold prepared in Example 1 was added onto a 24-well multiplate (No. 353047 manufactured by Nippon Becton Dickinson Co. Ltd.), and the mixture was rapidly stirred such that it was distributed onto the plate as a whole, so as to produce a uniform biological scaffold at the bottom of the plate (n = 3). 20 minutes later, the bottom of the plate was washed with a phosphate buffered saline. Normal human fetal lung-derived fibroblasts HEL299 (HEL ATCC No. CCL137) with a cell number of 2 x $10^4$ which were suspended in MEM-E medium (12-102-504, Dainippon Pharmaceutical Co., Ltd.) containing 4 mM glutamine and 10% fetal bovine serum were added thereto. The cells were cultured at 37°C in a carbon dioxide incubator containing 5% $CO_2$ (BNA121D manufactured by Tabai-Espec Corp.) for 72 hours. After completion of the culture, non-attached cells were eliminated by washing with a phosphate buffered saline that had previously been warmed to 37°C. 1 ml of a 0.25% trypsin solution (15050-065, Invitrogen Corp.) was added to each well, and the plate was then left at rest at 37°C in a carbon dioxide incubator. 30 minutes later, normal human fetal lung-derived fibroblasts were recovered. Thereafter, the number of cells was counted based on a calibration curve relating to the simultaneously prepared normal human fetal lung-derived fibroblasts, using CyQUANT Cell Proliferation Assay Kit (manufactured by Molecular Probes Inc.) in accordance with the procedure manual provided from the manufacturer. The number of the normal human fetal lung-derived fibroblasts cultured on the fibrin-containing biological scaffold produced in Example 1 was 12.5 ± 0.3 × $10^4$ (mean value of n = 3 ± standard deviation). The obtained number of the cells were significantly greater than that of the cells cultured on a fibrin-containing biological scaffold produced using a fibrinogen composition with a recovery rate of 40.5% produced in Comparative example 1, as described later.

Likewise, the activity of stimulating the growth of the normal human fetal lung-derived fibroblasts cultured on the fibrin-containing biological scaffold produced in Example 2 was measured. As a result, it was found to be 8.4 ± 0.3 × $10^4$ (mean value of n = 3 ± standard deviation).

Likewise, the activity of stimulating the growth of the normal human fetal lung-derived fibroblasts (HEL) cultured on the fibrin-containing biological scaffold produced in Comparative example 1 was measured. As a result, it was found to be 6.3 ± 0.2 × $10^4$ (mean value of n = 3 ± standard deviation).

Likewise, the activity of stimulating the growth of the normal human fetal lung-derived fibroblasts cultured on the fibrin-containing biological scaffold produced in Comparative example 2 was measured. As a result, it was found to be 4.1 ± 0.2 x $10^4$ (mean value of n = 3 ± standard deviation).

The recovery rate of fibrinogen in each of the fibrinogen concentrates obtained in Examples 1 and 2 and Comparative examples 1 and 2, and the activity of the fibrin-containing biological scaffold produced from each fibrinogen concentrate to stimulate the growth of the normal human fetal lung-derived fibroblasts are summarized in the following Table 1.

Table 1

|  | Recovery rate of fibrinogen (%) | Fibroblast growth-stimulating activity (The number of cells: x $10^4$ cells) |
|---|---|---|
| Example 1 | 20.3 | $12.5 \pm 0.3$ |
| Example 2 | 28.6 | $8.4 \pm 0.3$ |
| Comparative example 1 | 40.5 | $6.3 \pm 0.2$ |
| Comparative example 2 | 12.5 | $4.1 \pm 0.2$ |

Test example 2: Adhesive ability to fibroblasts

The ability of the biological scaffold produced in Example 1 to adhere to fibroblasts was measured. 0.5 ml of the fibrinogen concentrate produced in Example 1 was rapidly mixed with a human plasma thrombin solution (T8885 manufactured by Sigma-Aldrich Inc.) (5.0 NIH units/ml) containing 50 mM calcium chloride (C5080 manufactured by Sigma-Aldrich Inc.) with equal volume, so as to produce a fibrin-containing biological scaffold (n = 3) on a 24-well multiplate (No. 353047 manufactured by Nippon Becton Dickinson Co. Ltd.). 20 minutes later, the fibrin-containing biological scaffold was washed with a phosphate buffered saline. Normal human skin fibroblasts (NHDF-Neo manufactured by Takara Bio Inc.) with a cell number of 2 x $10^4$ which were suspended in D-MEM medium (manufactured by Invitrogen Corp.) containing 4 mM glutamine and 2% fetal bovine serum were added thereto. The cells were cultured at 37°C in a 5% $CO_2$ incubator for 60 minutes. 60 minutes later, the plate was removed from the incubator and washed with a phosphate buffered saline that had previously been warmed to 37°C. The attached cells were immobilized with methanol and subjected to Giemsa staining, and then it was observed with an optical microscope. As a result, it was confirmed that sufficiently developed normal human skin fibroblasts were adhered on the biological scaffold produced in Example 1.

Test example 3: Migration ability to fibroblasts

The migration ability of the fibrin-containing biological scaffold produced in Example 1 to fibroblasts was measured. 0.5 ml of the fibrinogen concentrate produced in Example 1 was rapidly mixed with a human plasma thrombin solution (T8885 manufactured by Sigma-Aldrich Inc.) (5.0 NIH units/ml) containing 50 mM calcium chloride (C5080 manufactured by Sigma-Aldrich Inc.) with equal volume, so as to produce a fibrin-containing biological scaffold (n = 3) on a 24-well multiplate (No. 353047 manufactured by Nippon Becton Dickinson Co. Ltd.). 20 minutes later, the fibrin-containing biological scaffold was washed with a phosphate buffered saline, and at the bottom of the 24-well multiplate (No. 353047 manufactured by Nippon Becton Dickinson Co. Ltd.), a fibrin-containing biological scaffold was formed. D-MEM medium (manufactured by Invitrogen Corp.) containing 4 mM glutamine and 2% fetal bovine serum was added thereto. A migration assay chamber (353431 manufactured by Nippon Japan Becton Dickinson Co. Ltd.) equipped with a filter coated with collagen was placed on the plate, and 2 x $10^4$ normal human skin fibroblasts (NHDF-Neo manufactured by Takara Bio Inc.) were added thereto. The cells were cultured at 37°C in a 5% $CO_2$ incubator. 2 hours later, cells migrating to the back of the filter was subjected to Giemsa staining and then observed with an optical microscope. As a result, it was found that the biological scaffold produced in Example 1 exhibited strong migration ability.

Test example 4: Cell growth-stimulating activity to bone marrow mesenchymal stem cells

The cell growth-stimulating activity of the biological scaffold produced in Example 1 to bone marrow mesenchymal stem cells was measured. 0.5 ml of the fibrinogen concentrate produced in Example 1 was rapidly mixed with a human plasma thrombin solution (T8885 manufactured by Sigma-Aldrich Inc.) (5.0 NIH units/ml) containing 50 mM calcium chloride (C5080 manufactured by Sigma-Aldrich Inc.) with equal volume. The mixture was added onto a 24-well multiplate (No. 353047 manufactured by Nippon Becton Dickinson Co. Ltd.), and it was rapidly stirred such that it was distributed onto the plate as a whole, so as to produce a uniform biological scaffold at the bottom of the plate. Subsequently, the bottom of the plate was washed with a phosphate buffered saline. 2.5 x $10^4$ normal human bone marrow-derived mesenchymal stem cells (PT034 manufactured by Takara Bio Inc.) which were suspended in D-MEM medium containing 4 mM glutamine and 2% fetal bovine serum were added thereto. The cells were cultured at 37°C in a carbon dioxide incubator containing 5% $CO_2$ for 72 hours. As a control, the same number of bone marrow mesenchymal stem cells were cultured on a plate without scaffold and used. After completion of the culture, dead cells were eliminated by washing with a phosphate buffered saline that had previously been warmed to 37°C. 1 ml of a 0.25% trypsin solution (15050-065, Invitrogen Corp.) was added to each well, and the plate was then left at rest at 37°C in a carbon dioxide incubator. 30 minutes later, the bone marrow mesenchymal stem cells were recovered. Thereafter, the number of cells was counted based on a calibration curve relating to the simultaneously prepared bone marrow mesenchymal stem cells, using CyQUANT Cell Proliferation Assay Kit (manufactured by Molecular Probes Inc.) in accordance with the procedure manual provided from the manufacturer. As a result, it was found that the biological scaffold produced in Example 1 exhibited higher cell growth-stimulating activity to human bone marrow mesenchymal stem cells, than that growing in the plate having no scaffold used as a control.

Test example 5: Cell growth-stimulating activity to human primary hepatic parenchymal cells

The cell growth-stimulating activity of the biological scaffold produced in Example 1 to human primary hepatic parenchymal cells was measured. 0.5 ml of the fibrinogen concentrate produced in Example 1 was rapidly mixed with a human plasma thrombin solution (T8885 manufactured by Sigma-Aldrich Inc.) (5.0 NIH units/ml) containing 50 mM calcium chloride (C5080 manufactured by Sigma-Aldrich Inc.) with equal volume. The mixture was added onto a 24-well multiplate (No. 353047 manufactured by Nippon Becton Dickinson Co. Ltd.), and it was rapidly stirred such that it was distributed onto the plate as a whole, so as to produce a uniform biological scaffold at the bottom of the plate.
Subsequently, the bottom of the plate was washed with a phosphate buffered saline. 2.5 x $10^4$ normal human hepatocytes (C2591 manufactured by Takara Bio Inc.) which were suspended in D-MEM medium containing 4 mM glutamine and 2% fetal bovine serum were added thereto. The cells were cultured at 37°C in a carbon dioxide incubator containing 5% $CO_2$ for 72 hours. As a control, the same number of normal human hepatocytes were cultured on a plate having no scaffold and used. After completion of the culture, non-attached cells were eliminated by washing with a phosphate buffered saline that had previously been warmed to 37°C. 1 ml of a 0.25% trypsin solution (15050-065, Invitrogen Inc.) was added to each well, and the plate was then left at rest at 37°C in a carbon dioxide incubator. 30 minutes later, the hepatic parenchymal cells were recovered. Thereafter, the number of cells was counted using Cy-QUANT Cell Proliferation Assay Kit (manufactured by Molecular Probes Inc.) in accordance with the procedure manual provided from the manufacturer. As a result, it was found that the biological scaffold produced under the conditions applied in Example 1 exhibited cell growth-stimulating activity to human hepatic parenchymal cells, which was higher than that growing in the plate having no scaffold used as a control.

Example 3

A fibrinogen concentrate was prepared from human plasma by a method equivalent to that in Example 1 (improved cryo method) and the hollow fiber membrane method.
50 ml of pooled plasma collected from healthy subjects (derived from multiple donors) [which had been prepared by centrifuging peripheral blood added with 56 ml of CPD as an anticoagulant (Citrate-phosphate-dextrose C7165 manufactured by Sigma-Aldrich Inc.) to 400 ml of the blood] was transferred into a 50-ml centrifuge tube (No. 352070 manufactured by Nippon Becton Dickinson Co. Ltd.). The centrifuge tube was then left at rest in a freezer (EEV-204N manufactured by Whirlpool Corp.) at -27°C for 30 minutes, so as to freeze it. Subsequently, the centrifuge tube containing frozen plasma was transferred into a refrigerated centrifuge, the chamber temperature of which had been set at -2.5°C (No.3740 manufactured by KUBOTA Corp.), and it was then left for 30 minutes. Subsequently, the centrifuge tube was transferred into another refrigerated centrifuge, the chamber temperature of which had previously been set at 12°C, and it was left for 30 minutes. Thereafter, the tube was left at rest in a refrigerator (Medicool MPR-510 manufactured by SANYO Electric Co. Ltd, 4°C) for 30 minutes. Finally, centrifugation (1,000 g x 15 minutes, 1°C) was carried out, and the supernatant was removed, so as to obtain a fibrinogen concentrate. Fibrinogen was quantified using a commercially available fibrinogen measurement kit (Pacific Hemostasis kit, Fisher Scientific International) that was based on the thrombin time method. Measurement was carried out in accordance with the procedure manual provided from the manufacturer. The amount of fibrinogen recovered was 14.3 mg. The amount of fibrinogen contained in plasma as a starting material was measured to be 86.5 mg. Thus, the recovery rate was 16.5% (14.3/86.5 $\times$ 100 = 16.5).
A fibrinogen concentrate was prepared using a hollow fiber membrane produced from EVAL as a material (EC-50W manufactured by Kawasumi Laboratories, Inc.; cutoff molecular weight: 300,000 daltons). The fibrinogen concentrate was prepared by the Aspirate method. Five hundreds of 20-cm fibers were unified to prepare a bundle. Two such bundles were prepared. First, a bundle was immersed in a vessel containing plasma, and the plasma was aspirated from the inner portion of the hollow fiber, using a peristaltic pump connecting to the inner portion of the hollow fiber. 100 ml of the plasma was concentrated to 25 ml. Thereafter, another bundle was immersed, and the plasma was aspirated, so that the concentrated plasma was further concentrated to 8 ml. The time required for the concentration operation was 6 minutes for the first bundle, and 11 minutes for the second bundle. Thus, it took 17 minutes in total for the concentration operations. The concentration of fibrinogen in the concentrate was 8.3 mg/ml. Since the concentration of fibrinogen in the starting material plasma was 1.73 mg/ml, the concentration rate was calculated to be 4.8 times. The recovery rate was 38%.

Test example 6: Cell growth-stimulating activity to human fetal lung-derived fibroblasts (HEL)

The activity of the biological scaffold produced in Example 3 to human fetal lung-derived fibroblasts (HEL) was measured in terms of cell growth-stimulating activity. 0.3 ml each of the human fibrinogen concentrates produced in Example 3 (adjusted with medium thereby resulting in a fibrinogen concentration of 5.63 mg/ml) was added to a 12-well multiplate (No. 353043 manufactured by Nippon Becton Dickinson Co. Ltd.). 0.02 ml of an HEL cell suspension containing 20,000 HEL cells was further added thereto. 0.3 ml of 31.3 NIH units/ml thrombin solution (a solution attached to Tisseel, a biological tissue adhesive manufactured by Baxter Corp., was diluted and used) was added to the plate such that it was rapidly distributed onto the plate as a whole, and the plate was then stirred. It was left at rest at 37°C in a carbon dioxide incubator containing 5% $CO_2$ (BNA121D manufactured by Tabai-Espec Corp.) to promote a gelation, thereby producing a fibrin scaffold in which HEL cells were embedded. 60 minutes later, D-MEM medium (manufactured by Invitrogen Corp) containing 167 μg/ml aprotinin (A4529 manufactured by Sigma-Aldrich Inc.) and fetal bovine serum with a final concentration of 10% was added thereto, and the cells were cultured in the same above incubator for 96 hours. Cell growth was assayed by colorimetry using CellTiter96 Aqueous One Solution Cell Proliferation Assay kit (Promega Corp.). Measurement was carried out in accordance with the procedure manual provided from the manufacturer. The measurement results regarding the cell growth test were shown with a relative value, which was obtained, when the number of cells (an absorbance at a wavelength of 490 nm) obtained using, as a scaffold, Tisseel (a biological tissue adhesive manufactured by Baxter Corp.) that was adjusted to have the same fibrinogen concentrate, was set at 1.00. As shown in Table 2, all the biological scaffolds produced by the methods applied in Example 3 exhibited cell growth-stimulating activity higher than that obtained in the case of using Tisseel as a scaffold. Thus, it was confirmed that these biological scaffolds have higher biological scaffold activity than control.

Table 2

| Method for producing scaffold | Cell growth-stimulating activity (the number of cells counted when Tisseel was used: 1.00) |
| --- | --- |
| Hollow fiber membrane method | 2.82 |
| Improved cryo method | 1.89 |
| Control (Tisseel) | 1.00 |

Example 4

A fibrinogen concentrate was prepared from bovine plasma by a method equivalent to that in Example 1 (improved cryo method) and the hollow fiber membrane method.

50 ml of bovine plasma [which had been prepared by centrifuging peripheral blood added with 56 ml of CPD as an anticoagulant (Citrate-phosphate-dextrose C7165 manufactured by Sigma-Aldrich Inc.) to 400 ml of the blood] was transferred into a 50-ml centrifuge tube (No. 352070 manufactured by Nippon Becton Dickinson Co. Ltd.). The centrifuge tube was then left at rest in a freezer (EEV-204N manufactured by Whirlpool Corp.) at -27°C for 30 minutes, so as to freeze it. Subsequently, the centrifuge tube containing frozen plasma was transferred into a refrigerated centrifuge, the chamber temperature of which had been set at -2.5°C (3740 manufactured by KUBOTA Corp.), and it was then left for 30 minutes. Subsequently, the centrifuge tube was transferred into another refrigerated centrifuge, the chamber temperature of which had previously been set at 12°C, and it was left for 30 minutes. Thereafter, the tube was left at rest in a refrigerator (Medicool MPR-510 manufactured by SANYO Electric Co. Ltd, 4°C) for 30 minutes. Finally, centrifugation (1,000 g x 15 minutes, 1°C) was carried out, and the supernatant was removed, so as to obtain a fibrinogen concentrate. Fibrinogen was quantified using a commercially available fibrinogen measurement kit (Pacific Hemostasis kit, Fisher Scientific International) that was based on the thrombin time method. Measurement was carried out in accordance with the procedure manual provided from the manufacturer. The amount of fibrinogen recovered was 18.7 mg. The amount of fibrinogen contained in plasma as a starting material was measured to be 94.1 mg. Thus, the recovery rate was 19.9% (18.7/94.1 $\times$ 100 = 19.9).

A fibrinogen concentrate was prepared using a hollow fiber membrane. The fibrinogen concentrate was prepared by the Discard method. The following materials were used: (1) hydrophilized polysulfone (cutoff molecular weight: 300,000 daltons); (2) EVAL (cutoff molecular weight: 300,000 daltons); (3) PAN (polyacrylonitrile; cutoff molecular weight: 200,000 daltons); and (4) CDA (cellulose diacetate; cutoff molecular weight: 350,000 daltons). Two thousands of fibers were filled in a small module with a length of 10 cm and a diameter of 3 cm, followed by molding. The ratio Bin/Bout was set at 10, and 100 ml of plasma was treated. The amount of plasma treated was 10 ml/min. The treating time was 10 minutes. With respect to 1.7 mg/ml that was a fibrinogen concentration in the starting material plasma, fibrinogen concentrates each having a concentration of (1) 7.2 mg/ml, (2) 8.1 mg/ml, (3) 6.5 mg/ml, and (4) 5.9 mg/ml, could be obtained.

Test example 7: Cell growth-stimulating activity to bovine trachea-derived fibroblasts (CCL-44)

The activity of the biological scaffold produced in Example 4 to bovine trachea-derived fibroblasts (ATCC No. CCL-44) was measured in terms of cell growth-stimulating activity. 0.3 ml each of various types of bovine fibrinogen concentrates produced in Example 4 (adjusted with medium thereby resulting in a fibrinogen concentration of 5.63 mg/ml) was added to a 12-well multiplate (No. 353043 manufactured by Nippon Becton Dickinson Co. Ltd.). 0.02 ml of a CCL-44 cell suspension containing $2 \times 10^4$ CCL-44 cells was further added thereto. 0.3 ml of a 31.3 NIH units/ml thrombin solution (a solution attached to Tisseel, a biological tissue adhesive manufactured by Baxter Corp., was diluted and used) was added to the plate such that it was rapidly distributed onto the plate as a whole, and the plate was then stirred. It was left at rest at 37°C in a carbon dioxide incubator containing 5% $CO_2$ (BNA121D manufactured by Tabai-Espec Corp.) to promote a gelation, thereby producing a fibrin-containing biological scaffold in which CCL-44 cells were embedded. 60 minutes later, Eagle's MEM medium (manufactured by Invitrogen Inc.) containing 167 µg/ml aprotinin (A4529 manufactured by Sigma-Aldrich Inc.) and fetal bovine serum with a final concentration of 10% was added thereto, and the cells were cultured in the same above incubator for 72 hours. Cell growth was assayed by colorimetry using CellTiter96 Aqueous One Solution Cell Proliferation Assay kit (Promega Corp.). Measurement was carried out in accordance with the procedure manual provided from the manufacturer. The measurement results regarding the cell growth test were shown with a relative value, which was obtained, when the number of cells (an absorbance at a wavelength of 490 nm) obtained using, as a scaffold, Tisseel that was adjusted to the same fibrinogen concentrate, was set at 1.00. As shown in Table 3, all the biological scaffolds produced by the methods applied in Example 4 exhibited cell growth-stimulating activity higher than that obtained in the case of using Tisseel as a scaffold. Thus, it was confirmed that these biological scaffolds have higher biological scaffold activity than control.

Table 3

| Cell growth-stimulating activity to CCL-44 cells | |
|---|---|
| Method for producing scaffold | Cell growth-stimulating activity (the number of cells counted when Tisseel was used: 1.00) |
| Hollow fiber membrane method (PS) | 2.10 |
| Hollow fiber membrane method (EVAL) | 2.00 |
| Hollow fiber membrane method (PAN) | 2.00 |
| Hollow fiber membrane method (CDA) | 2.33 |
| Improved cryo method | 1.88 |
| Control (Tisseel) | 1.00 |

Example 5: Preparation of activated platelet-released and leukocyte-released substances, and measurement of cell growth-stimulating activity

As stated below, a filter where platelets and leukocytes were captured was prepared.
50 ml of fresh peripheral blood [to which 7.0 ml of CPD was added as an anticoagulant] was collected from a healthy subject. A polyethylene terephthalate non-woven (Asahi Kasei Corp.; mean fiber diameter: 2.6 µm, weight per unit area: 90 g/m², thickness: 0.38 mm) was punched into 25 mm in diameter. Three of these non-wovens were laminated and filled into a column (PP-25, Advantech Co.Ltd.). Thereafter, 10 ml of the peripheral blood was passed through the column. The blood was treated, and 10 ml of a phosphate buffered saline was passed through the column for washing. This operation was carried out 3 times, so that it was washed with total 30 ml of the phosphate buffered saline. The time required for this operation was 15 minutes or shorter in each operation. The number of platelets and the number of leukocytes were counted with an automatic cell counter (Beckman Coulter Inc.) before and after the treatment. The rates of the leukocytes, erythrocytes, and platelets captured by the non-woven filter layer are as follows.
the capture rate of leukocytes: 85.3%;
the capture rate of erythrocytes: 8.3%;

the capture rate of platelets: 94.5%

Subsequently, 1 ml of a phosphate buffered saline containing 0.5 NIH units/ml thrombin was passed through the column, so as to recover platelet- and leukocyte-released substances. The recovered substance was conserved at -80°C in a frozen state until it was used.

Test example 8

The activated platelet- and activated leukocyte-released substances prepared in Example 5 were added to fibroblasts, and the cell growth-stimulating activity thereof was examined. 2 x $10^4$ normal human fetal lung-derived fibroblasts were inoculated on a 12-well cell culture plate (353043 manufactured by Nippon Becton Dickinson Co. Ltd.) (the medium amount: 1 ml/well). The culture medium was prepared by adding 1 mM sodium pyruvate (manufactured by ICN Inc.), 2 mM non-essential amino acid (manufactured by ICN Inc.), and 2 mM L-glutamine to DMEM (manufactured by Invitrogen Inc.), and further adding 10% fetal bovine serum (StemCell Technologies Inc.) thereto at final concentration. As controls, 100 µl of 0.5 NIH units/ml thrombin solution or phosphate buffered saline was added to culture system. 100 µl of the recovery solution prepared in Example 5 was added thereto, and the cells were cultured in a 5% $CO_2$ incubator for 3 days. Thereafter, the number of cells was counted. As shown in Table 4, the recovery solution containing the activated platelet- and activated leukocyte-released substances prepared in Example 5 exhibited fibroblast growth-stimulating activity that was higher than that of the phosphate buffered saline or the thrombin solution used as controls.

Table 4

| Fibroblast growth-stimulating activity of solution recovered with platelet and leukocyte capturing filter | |
|---|---|
| Additives | Number of cells (x $10^4$ cells) |
| Recovered solution (100 µl) | 9.6 ± 0.2 |
| Control (thrombin solution (100 µl)) | 8.2 ± 0.6 |
| Control (phosphate buffered saline (100 µl)) | 7.1 ± 0.2 |

Test example 9

The activated platelet- and activated leukocyte-released substances prepared in Example 5 were added to vascular endothelial cells, and the cell growth-stimulating activity thereof was examined. 2 x $10^4$ normal human umbilical vein endothelial cells were inoculated on a 12-well cell culture plate (353043 manufactured by Nippon Becton Dickinson Co. Ltd.) (the medium amount: 1 ml/well). The culture medium was prepared by adding fetal bovine serum (StemCell Technologies Inc.) to serum-free basal medium for culturing human vascular endothelial cells (Cat. No. 11111-044 manufactured by Invitrogen Inc.), resulting in a final concentration of 5%. As controls, 100 µl of 0.5 NIH units/ml thrombin solution or a phosphate buffered saline was added to culture system. 100 µl of the recovery solution prepared in Example 5 was added to culture system, and the cells were cultured in a 5% $CO_2$ incubator for 3 days. Thereafter, the number of cells was counted. As shown in Table 5, the recovery solution containing the activated platelet- and activated leukocyte-released substances prepared in Example 5 exhibited vascular endothelial cell growth-stimulating activity that was higher than those of the phosphate buffered saline or the thrombin solution used as controls.

Table 5

| Vascular endothelial cell growth-stimulating activity of solution recovered with platelet and leukocyte capturing filter | |
|---|---|
| Additives | Number of cells (x $10^4$ cells) |
| Peripheral blood eluant (100 µl) | 1.10 ± 0.05 |
| Control (thrombin solution (100 µl)) | 1.01 ± 0.06 |
| Control (phosphate buffered saline) (100 µl)) | 0.71 ± 0.09 |

Example 6

An ethylene/vinyl alcohol copolymer was used as a hollow fiber membrane. This hollow fiber membrane had an internal diameter of 175 µm (EC-50W manufactured by Kawasumi Laboratories, Inc.).
Two thousands of fibers were filled in a small module with a length of 10 cm and a diameter of 3 cm, followed by molding. The Discard method was applied herein. The ratio Bin/Bout was set at 10, and 50 ml of plasma was treated. As a result, 7.7 mg/ml fibrinogen concentrate could be obtained. The amount of plasma treated was 10 ml/min. The treating time was 5 minutes. A fibrinogen concentration in the starting material plasma was 1.7 mg/ml. Accordingly, the concentration rate was found to be 4.5 times.

Example 7

The Aspirate method was applied using the same hollow fiber membrane as used in Example 6. Five hundreds of 20-cm fibers were unified to prepare a bundle, and both ends were allowed to adhere. The bundle was immersed in a vessel containing plasma, and the plasma was aspirated from the inner portion of the hollow fiber, using a peristaltic pump connecting to the inner portion of the hollow fiber. When 200 ml of the plasma was concentrated to 40 ml, the fibrinogen concentration became 8.9 mg/ml. Since the concentration of fibrinogen in the starting material plasma was 1.9 mg/ml, the concentration rate was calculated to be 4.7 times.

Example 8

As with Example 7, the Aspirate method was applied herein. Five hundreds of 20-cm fibers were unified to prepare a bundle. Four such bundles were prepared. First, a bundle was immersed in a vessel containing plasma, and the plasma was aspirated from the inner portion of the hollow fiber, using a peristaltic pump connecting to the inner portion of the hollow fiber. 200 ml of the plasma was concentrated to 60 ml. Thereafter, another bundle was immersed, and the plasma was aspirated, so that the concentrated plasma was further concentrated to 20 ml. Thereafter, the third bundle was used, so that the plasma was concentrated to 10 ml, and the fourth bundle was used so that the plasma was further concentrated to 5 ml. The time required for concentration using 4 bundles was 43 minutes. The concentration of fibrinogen became 16.3 mg/ml. Since the concentration of fibrinogen in the starting material plasma was 1.8 mg/ml, the concentration rate was calculated to be 9 times.

Example 9

Using a plasma separation column and a plasma filtration column with the structures shown in Figure 7 and an activated thrombin plasma preparation device with the structure shown in Figure 8, the blood treatment device shown in Figure 6 was fabricated.
In Figure 7, the blood or plasma introduced into a blood or plasma entrance (21), was passed through a hollow fiber membrane (24), and was thereby separated into fractions containing large quantities of blood cells eliminated from plasma and the plasma, or into concentrated plasma and filtrated plasma. These were then discharged from exists (22) and (23), separately.
A polyethylene hollow fiber membrane (the maximum pore size: 0.3 $\mu$m) coated with ethylene-vinyl alcohol was used as a plasma separation column. A cellulose diacetate hollow fiber membrane (mean pore size: 0.1 $\mu$m; cutoff molecular weight: 90 kDa) was used as a plasma filtration device.
500 ml of CPD-added bovine whole blood was introduced into a plasma separation column at a constant flow rate of 100 ml/min., so as to obtain 200 ml of plasma. The obtained plasma was introduced into a plasma filtration column at a constant flow rate of 25 ml/min., and the plasma passed through the membrane (hereinafter referred to as filtrated plasma) was discharged from an exist disposed on the lateral face of the column at a constant flow rate of 25 ml/min. The plasma was stored in an activated thrombin plasma preparation device. At the same time, plasma concentrated without passing through a membrane (hereinafter referred to as concentrated plasma) was discharged at a constant flow rate of 1 ml/min., and it was stored.
As a result of such treatment, 158 ml of the filtrated plasma and 8 ml of the concentrated plasma were obtained. The concentration of fibrinogen in the plasma before introduction into the plasma filtration column was 2.8 mg/ml, and the concentration of fibrinogen in the filtrated plasma was less than 1 mg/ml. The concentration of fibrinogen in the concentrated plasma was 42 mg/ml.
The filtrated plasma was mixed with a calcium-added physiological saline (33) and silicon beads (34) in an activated thrombin plasma preparation device shown in Figure 8 containing a plasma storage bag (32) made from vinyl chloride, which was filled with the calcium-added physiological saline solution and the silicon beads. Thereafter, the mixture was filtrated through a filter for eliminating the silicon beads. The filtrated plasma was stored as activated thrombin plasma.
8 ml of the concentrated plasma was mixed with 5 ml of the activated thrombin plasma, so as to produce a fibrin-containing biological scaffold. The time required from the mixing of both solutions to coagulation was less than 2 seconds. In addition, the produced fibrin-containing biological scaffold was conserved at room temperature for 24 hours. As a result, no changes were observed by naked eyes. From these results, it was confirmed that a stable fibrin-containing biological scaffold with excellent coagulation ability can be produced by the method of the present invention.

INDUSTRIAL APPLICABILITY

The present invention can provide a biological scaffold with high performance that is suitable for tissue regeneration. Since the fibrin-containing biological scaffold of the present invention has activity of stimulating the growth of various types of cells, it is useful for hemostasis due to adhesion and sealing of tissues, wound healing, the formation of artificial bones, skins, organs, etc., treatments after liver transplantation and the like, the production of a large amount of albumin, researches regarding drug metabolism, etc.

Moreover, the present invention provides a method for rapidly and simply producing a safe and stable fibrin-containing biological scaffold having a low risk of infection, and a production system thereof.

All of the contents of Japanese Patent Application Nos. 2002-243923 and 2002-244294, which are priority documents of the present application, are incorporated herein by reference as a part of the disclosure of the present specification.

**Claims**

1. A fibrin-containing biological scaffold, which is **characterized in that** it comprises a mixture consisting of a fibrinogen concentrate obtained from human plasma by a short-time rough purification step and a fibrinogen activator, when a fibrin composition is used in the regeneration of human tissues and the cell growth.

2. The fibrin-containing biological scaffold according to claim 1, wherein said fibrinogen concentrate is obtained by a method comprising steps of cooling the plasma for a short time, rapidly thawing the plasma, and recovering the fibrinogen concentrate.

3. The fibrin-containing biological scaffold according to claim 1 or 2, wherein said fibrinogen concentrate is obtained from the plasma by precipitation, resulting in a recovery rate of fibrinogen within the range between 15% and 32%.

4. The fibrin-containing biological scaffold according to any one of claims 1 to 3, wherein said fibrinogen concentrate is obtained by a method comprising steps of cooling the plasma for 10 to 60 minutes, thawing the plasma for 15 to 60 minutes, and recovering the fibrinogen concentrate.

5. The fibrin-containing biological scaffold according to claim 3 or 4, wherein the cooling step is carried out at a temperature between -20°C and -40°C and the thawing step is carried out at a temperature between -10°C and +15°C.

6. The fibrin-containing biological scaffold according to claim 1, wherein said fibrinogen concentrate is obtained from human plasma using a plasma component fractionation device.

7. The fibrin-containing biological scaffold according to claim 6, wherein said plasma component fractionation device comprises therein a hollow fiber membrane used for fractionation of plasma components.

8. The fibrin-containing biological scaffold according to claim 7, wherein the material for said hollow fiber membrane is any one selected from the group consisting of hydrophilic polysulfone, EVAL (ethylene-vinyl alcohol copolymer), PAN (polyacrylonitrile), CDA (cellulose diacetate), and CTA (cellulose triacetate).

9. The fibrin-containing biological scaffold according to claim 8, wherein said material for the hollow fiber membrane is hydrophilic polysulfone or EVAL (ethylene-vinyl alcohol copolymer).

10. The fibrin-containing biological scaffold according to any one of claims 6 to 9, wherein the cutoff value of said hollow fiber membrane is between 80,000 daltons and 300,000 daltons.

11. The fibrin-containing biological scaffold according to any one of claims 6 to 9, wherein the cutoff value of said hollow fiber membrane is between 150,000 daltons and 400,000 daltons.

12. The fibrin-containing biological scaffold according to any one of claims 6 to 11, wherein said fibrinogen concentrate is obtained from the end of a hollow fiber by supplying human plasma to a hollow portion of the hollow fiber in a plasma component fractionation device, and allowing mainly liquid components to permeate from the inner surface of the hollow fiber to the outer surface thereof.

13. The fibrin-containing biological scaffold according to claim 12, wherein the ratio (Bin/Bout) between the amount (Bin) of the patient plasma supplied to said hollow portion per unit time and the amount (Bout) of fibrinogen concentrate collected from the end of the hollow fiber per unit time is within the range between 2 and 20.

14. The fibrin-containing biological scaffold according to claim 12 or 13, wherein said Bin/Bout ratio is within the range between 5 and 10.

15. The fibrin-containing biological scaffold according to any one of claims 6 to 14, wherein said fibrinogen concentrate is obtained by allowing human plasma to come into contact with the outer surface of a hollow fiber in a plasma component fractionation device, and allowing mainly liquid components to permeate from the outer surface of the hollow fiber to the inner surface thereof, thereby concentrating the plasma allowed to be contacted with the outer portion of the hollow fiber.

16. The fibrin-containing biological scaffold according to claim 15, wherein, when liquid components are allowed to permeate, the hollow portion of the hollow fiber is depressurized, and the liquid components are allowed to permeate by aspiration from the outer portion of the hollow fiber to the inner portion thereof.

17. The fibrin-containing biological scaffold according to claim 15 or 16, wherein the ratio (Cinitial/Cend) between the amount (Cinitial) of plasma that is allowed to come into contact with the outer surface of said hollow fiber and the amount (Cend) of a fibrinogen concentrate obtained by allowing mainly liquid components to permeate and by concentrating the plasma that is allowed to be contacted with the outer surface of the hollow fiber is within the range between 2 and 20.

18. The fibrin-containing biological scaffold according to any one of claim 15 to 17, wherein said ratio Cinitial/Cend is within the range between 5 and 10.

19. The fibrin-containing biological scaffold according to any one of claims 15 to 18, wherein the amount of the patient plasma allowed to come into contact with the outer surface of said hollow fiber is between $5 \times 10^{-5}$ and $5 \times 10^{-4}$ $m^3$, the outer surface area of the hollow fiber allowed to come into contact with the plasma is between 0.001 and 1 $m^2$, and a differential pressure caused by aspiration is between 0.001 and 0.08 MPa.

20. The fibrin-containing biological scaffold according to any one of claims 1 to 19, wherein said fibrinogen activator is thrombin.

21. The fibrin-containing biological scaffold according to claim 20, wherein said thrombin is obtained from the blood of a single person, from which fibrinogen is obtained.

22. The fibrin-containing biological scaffold according to any one of claims 1 to 21, which is used in culture of cells selected from the group consisting of vascular endothelial cells, fibroblasts, keratinocytes, mesenchymal stem cells, osteocytes; osteoblasts, osteoclasts, liver cells, pancreatic cells, and hematopoietic stem cells.

23. The fibrin-containing biological scaffold according to any one of claims 1 to 22, which has a cell growth-stimulating activity that is greater than those in the cases of culturing the cells with no biological scaffold or with a purified fibrinogen concentrate as a control, when at least one type of cells selected from the group consisting of vascular endothelial cells, fibroblasts, keratinocytes, mesenchymal stem cells, osteocytes, osteoblasts, osteoclasts, liver cells, pancreatic cells, and hematopoietic stem cells is cultured.

24. A method for culturing cells or regenerating tissues, which comprises culturing cells using the fibrin-containing biological scaffold according to any one of claims 1 to 23.

25. The method according to claim 24, wherein said cells are selected from the group consisting of vascular endothelial cells, fibroblasts, keratinocytes, mesenchymal stem cells, osteocytes, osteoblasts, osteoclasts, liver cells, pancreatic cells, and hematopoietic stem cells.

26. The method according to claim 24 or 25, wherein the above cells are derived from a single person, from who plasma used as a starting material for a fibrinogen concentrate is collected.

27. The method according to any one of claims 24 to 26, wherein the culture is carried out in the presence of a substance that stimulates cell growth and/or differentiation.

28. The method according to claim 27, wherein said substance that stimulates cell growth and/or differentiation is a substance which is released from platelets.

29. The method according to claim 28, wherein said substance released from platelets is obtained by a method comprising the following steps:

(1) a step of allowing the whole blood to flow through a first-stage filter for giving passage to erythrocytes, platelets and plasma, and adsorbing leukocytes, so as to obtain fractions permeated through the filter;
(2) a step of allowing the permeated fractions obtained in (1) above to flow through a second-stage filter for adsorbing platelets and giving passage to erythrocytes, so as to obtain a filter on which platelets are adsorbed; and
(3) a step of allowing a recovery solution containing a platelet activator to flow through the filter obtained in (2) above, so as to obtain a solution containing an activated platelet-released substance.

30. The method according to claim 29, wherein said platelet activator is at least one substance selected from the group consisting of ATP, ADP, collagen, and thrombin.

31. The method according to claim 27, wherein said substance that stimulates cell growth and/or differentiation is a substance which is released from leukocytes.

32. The method according to claim 31, wherein said substance released from leukocytes is obtained by a method comprising the following steps:

(1) a step of allowing the whole blood to flow through a first-stage filter for giving passage to erythrocytes, platelets and plasma, and adsorbing leukocytes, so as to obtain a filter on which leukocytes are adsorbed; and
(2) a step of allowing a recovery solution containing a leukocyte activator to flow through the filter obtained in (1) above, so as to obtain a solution containing an activated leukocyte-released substance.

33. The method according to claim 27, wherein said substance that stimulates cell growth and/or differentiation is a mixture consisting of a substance released from platelets and a substance released from leukocytes.

34. The method according to claim 33, wherein said mixture consisting of a substance released from platelets and a substance released from leukocytes is obtained by a method comprising the following steps:

(1) a step of allowing the whole blood to flow through a filter for giving passage to erythrocytes and plasma and adsorbing platelets and leukocytes, so as to obtain a filter on which platelets and leukocytes are adsorbed; and
(2) a step of allowing a recovery solution containing a platelet activator and a leukocyte activator to flow through the filter obtained in (1) above, so as to obtain a solution containing an activated platelet-released substance and an activated leukocyte-released substance.

35. The method according to any one of claims 24 to 34, wherein the cells are obtained by allowing cells derived from a human to flow through a filter.

36. The method according to any one of claims 24 to 34, wherein the plasma used as a starting material for a fibrinogen concentrate is obtained by allowing human blood to flow through a filter.

37. A cell culture or regenerated tissue supported on a scaffold, which is obtained by the method according to any one of claims 24 to 36.

38. A method for promoting tissue regeneration, wherein the cell culture or regenerated tissue according to claim 37 is applied to damaged tissues, or is used as a graft.

**39.** A method for promoting tissue regeneration, which comprises a step of adding to damaged tissues a mixture obtained by mixing the biological scaffold according to any one of claims 1 to 23 and cells.

**40.** The method for promoting tissue regeneration according to claim 39, wherein said cells are at least one type of cells selected from the group consisting of vascular endothelial cells, fibroblasts, keratinocytes, mesenchymal stem cells, osteocytes, osteoblasts, osteoclasts, liver cells, pancreatic cells, and hematopoietic stem cells.

**41.** A concentration system for obtaining a fibrin-containing biological scaffold, which comprises the following means:

(1) a means for roughly purifying human plasma by a plasma component fractionation membrane;
(2) a means for introducing human plasma into the surface of said membrane; and
(3) a means for obtaining a fibrinogen concentrate from the surface of said membrane.

**42.** The system according to claim 41, which is **characterized in that** the cutoff value of said plasma component fractionation membrane is between 80,000 daltons and 300,000 daltons.

**43.** The system according to claim 41, which is **characterized in that** the cutoff value of said plasma component fractionation membrane is between 150,000 daltons and 400,000 daltons.

**44.** The concentration system according to claim 42 or 43, wherein said plasma component fractionation membrane is a hollow fiber membrane.

**45.** The system according to claim 44, which is **characterized in that** the material for said hollow fiber membrane is any one selected from the group consisting of hydrophilic polysulfone, EVAL (ethylene-vinyl alcohol copolymer), PAN (polyacrylonitrile), CDA (cellulose diacetate), and CTA (cellulose triacetate).

**46.** The system according to claim 45, wherein said material for the hollow fiber membrane is hydrophilic polysulfone or EVAL (ethylene-vinyl alcohol copolymer).

**47.** The concentration system according to any one of claims 41 to 46, wherein said introducing means is a liquid-supplying or liquid-aspirating device for introducing human plasma from one of flow ports provided on said fractionation device into the inner or outer membrane surface of the hollow fiber membrane and discharging it from another flow port.

**48.** The concentration system according to any one of claims 41 to 47, wherein said means for obtaining said concentrate is a means for storing the concentrate that is connected to one of the flow ports provided on said fractionation device.

**49.** The concentration system according to any one of claims 41 to 48, wherein said rough purification means is a plasma component fractionation device where both ends of a hollow fiber membrane built in a vessel are potted such that the inner portion of the hollow is communicated with the outer portion of the vessel.

**50.** The concentration system according to any one of claims 41 to 48, wherein said rough purification means is a plasma component fractionation device where one end of a hollow fiber membrane built in a vessel is potted such that the inner portion of the hollow is communicated with the outer portion of the vessel, and the other end is sealed.

**51.** A method for operating the concentration system according to claim 49, which comprises supplying human plasma to a hollow portion of the follow fiber in a plasma component fractionation device, allowing mainly liquid components to permeate from the inner surface of the hollow fiber to the outer surface thereof, and collecting a fibrinogen concentrate from the end of the hollow fiber.

**52.** The method for operating the concentration system according to claim 51, wherein the ratio (Bin/Bout) between the amount of the patient plasma supplied to said hollow portion per unit time (Bin) and the amount of fibrinogen concentrate collected from the end of the hollow fiber per unit time (Bout) is within the range between 2 and 20.

**53.** The method for operating the concentration system according to claim 52, wherein said Bin/Bout ratio is within the range between 5 and 10.

**54.** A method for operating the concentration system according to claim 50, which comprises allowing human plasma to come into contact with the outer surface of a hollow fiber in a plasma component fractionation device, allowing mainly liquid components to permeate from the outer surface of the hollow fiber to the inner surface thereof, and concentrating the plasma allowed to be contacted with the outer portion of the hollow fiber, so as to obtain a fibrinogen concentrate.

**55.** The method for operating the concentration system according to claim 54, which comprises allowing plasma to come into contact with the outer surface of a hollow fiber, and depressurizing the hollow portion of the hollow fiber when mainly liquid components are allowed to permeate from the outer surface of the hollow fiber to the inner surface thereof, so that said components are allowed to permeate by aspiration from the outer portion of the hollow fiber to the inner portion thereof.

**56.** The method for operating the concentration system according to claim 55, wherein said ratio Cinitial/Cend between the amount (Cinitial) of plasma that is allowed to come into contact with the outer surface of said hollow fiber and the amount (Cend) of a fibrinogen concentrate obtained by allowing mainly liquid components to permeate and concentrating the plasma that is allowed to be contacted with the outer surface of the hollow fiber is within the range between 2 and 20.

**57.** The method for operating the concentration system according to claim 54, wherein said ratio Cinitial/Cend is within the range between 5 and 10.

**58.** The method for operating the concentration system according to any one of claims 54 to 57, wherein the amount of the patient plasma allowed to come into contact with the outer surface of said hollow fiber is between $5 \times 10^{-5}$ and $5 \times 10^{-4}$ m$^3$, the outer surface area of the hollow fiber allowed to come into contact with the plasma is between 0.001 and 1 m$^2$, and a differential pressure caused by aspiration is between 0.001 and 0.08 MPa.

**59.** A system for producing a fibrin-containing biological scaffold, which comprises the following means:

(1) a means for fractionating human plasma by a plasma component fractionation membrane having a cutoff value between 80,000 daltons and 300,000 daltons, so as to separate a fibrinogen concentrate from the residual fractionated plasma;
(2) a means for recovering said fibrinogen concentrate and the residual fractionated plasma, separately;
(3) a means for producing fibrin glue from said fibrinogen concentrate; and
(4) a means for recycling the residual fractionated plasma.

**60.** A system for producing a fibrin-containing biological scaffold, which comprises the following means:

(1) a means for fractionating human plasma by a plasma component fractionation membrane having a cutoff value between 150,000 daltons and 400,000 daltons, so as to separate a fibrinogen concentrate from the residual fractionated plasma;
(2) a means for recovering said fibrinogen concentrate and the residual fractionated plasma, separately;
(3) a means for producing fibrin glue from said fibrinogen concentrate; and
(4) a means for recycling the residual fractionated plasma.

**61.** The system according to claim 59 or 60, wherein said human plasma is plasma which is collected by continuous extracorporeal circulation.

**62.** The system according to any one of claims 59 to 61, wherein said means for collecting human plasma has a means for separating plasma from the whole blood.

**63.** The system according to any one of claims 59 to 62, wherein said means for separating human plasma is gravity separation, centrifugation, or a membrane separation means.

**64.** The system according to any one of claims 59 to 63, wherein activated thrombin plasma is prepared from human plasma that is used to obtain a fibrinogen concentrate.

**65.** The system according to any one of claims 59 to 64, wherein an activated thrombin solution is obtained from said fibrinogen concentrate.

**66.** The system according to any one of claims 59 to 65, wherein the residual fractionated plasma obtained as a result of the fractionation by a plasma component fractionation membrane is used to prepare activated thrombin plasma.

**67.** The system according to any one of claims 59 to 66, wherein the residual fractionated plasma obtained as a result of the fractionation by a plasma component fractionation membrane is mixed with plasma-separated blood, from which plasma has been separated by a plasma separation means, and the mixture is then returned to a human body.

**68.** The system according to any one of claims 59 to 67, wherein said recovery system has a means for storing the obtained fibrinogen concentrate in the form of plasma containing a fibrinogen concentrate derived from a single donor.

**69.** The system according to any one of claims 59 to 68, wherein said means for producing fibrin glue has a means for mixing a fibrinogen concentrate, a fibrin stabilizing factor, and a fibrinogen activating factor.

**70.** A method for producing a fibrin-containing biological scaffold, which comprises the following steps:

(1) a step of fractionating human plasma by a plasma component fractionation membrane having a cutoff value between 80,000 daltons and 300,000 daltons, so as to separate a fibrinogen concentrate from the residual fractionated plasma;
(2) a step of recovering separately a high molecular weight fractionated plasma containing a large amount of fibrinogen concentrate and the residual fractionated plasma;
(3) a step of producing fibrin glue from said high molecular weight fractionated plasma containing a large amount of fibrinogen concentrate; and
(4) a step of recycling the residual fractionated plasma.

**71.** A method for producing a fibrin-containing biological scaffold, which comprises the following steps:

(1) a step of fractionating human plasma by a plasma component fractionation membrane having a cutoff value between 150,000 daltons and 400,000 daltons, so as to separate a fibrinogen concentrate from the residual fractionated plasma;
(2) a step of recovering separately a high molecular weight fractionated plasma containing a large amount of fibrinogen concentrate and the residual fractionated plasma;
(3) a step of producing fibrin glue from said high molecular weight fractionated plasma containing a large amount of fibrinogen concentrate; and
(4) a step of recycling the residual fractionated plasma.

**72.** The method according to claim 70 or 71, which comprises a step of collecting said human plasma by continuous extracorporeal circulation.

**73.** The method according to any one of claims 70 to 72, wherein said step of collecting human plasma comprises a step of separating plasma from the whole blood.

**74.** The method according to any one of claims 70 to 73, wherein said step of separating plasma is gravity separation, centrifugation, or a membrane separation step.

**75.** The method according to any one of claims 70 to 74, wherein the residual fractionated plasma obtained as a result of the fractionation by a plasma component fractionation membrane is mixed with plasma-separated blood, from which plasma has been separated by the plasma separation means, and the mixture is then returned to a human body.

**76.** The method according to any one of claims 70 to 75, wherein said recovery step comprises a step of storing the obtained fibrinogen concentrate in the form of fibrinogen concentrated plasma derived from a single donor.

**77.** The method according to any one of claims 70 to 76, wherein the step of producing fibrin glue comprises a step of mixing a high molecular weight fractionated plasma containing a large amount of fibrinogen, a fibrin stabilizing factor, and a fibrinogen activating factor.

Fig. 1

Fig. 2

Fig. 3

8

Fig. 4

1

9

Fig. 5

Fig. 6

Fig. 7

Fig. 8

## INTERNATIONAL SEARCH REPORT

| International application No. |
|---|
| PCT/JP03/10692 |

**A. CLASSIFICATION OF SUBJECT MATTER**
    Int.Cl$^7$ C12M3/00, A61L27/22, C12N5/06, C12N11/02

According to International Patent Classification (IPC) or to both national classification and IPC

**B. FIELDS SEARCHED**

Minimum documentation searched (classification system followed by classification symbols)
    Int.Cl$^7$ A61K35/14-16, A61L27/00, A61M1/16-18, C12M3/00

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 00/09018 A1 (FIBROGEN, INC.), 24 February, 2000 (24.02.00), & EP 1105051 A1 & JP 2002-524110 A | 1-37,41-71 |
| Y | WO 97/44135 A1 (THERMOGENESIS CORP.), 27 November, 1997 (27.11.97), & EP 901405 A1 & US 6077447 A1 & JP 2001-513073 A | 1-37 |
| Y | JP 6-141844 A (Fuji Photo Film Co., Ltd.), 24 May, 1994 (24.05.94), (Family: none) | 1-37,41-71 |
| Y | JP 11-246420 A (Sekisui Chemical Co., Ltd.), 14 September, 1999 (14.09.99), (Family: none) | 1-37,63 |

| ☒ | Further documents are listed in the continuation of Box C. | ☐ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 November, 2003 (28.11.03) | 09 December, 2003 (09.12.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.

PCT/JP03/10692

C (Continuation). DOCUMENTS CONSIDERED TO BE RELEVANT

| Category* | Citation of document, with indication, where appropriate, of the relevant passages | Relevant to claim No. |
|---|---|---|
| Y | Yoshiki TAKAMATSU, "Tensei Fibrinogen Ketsubosho/ Ijo Fibrinogen Kessho", separate volume Japanese Journal of Clinical Medicine, Ryoikibetsu Shokogun Series No.21 Ketsueki Shokogun II, Nippon Rinshosha, 30 September, 1998 (30.09.98), page 449 | 6-19,41-71 |
| Y | JP 58-206757 A (Asahi Medical Co., Ltd.), 02 December, 1983 (02.12.83), (Family: none) | 6-19,29,30, 32,34,36, 41-71 |
| Y | JP 2002-186667 A (Toyobo Co., Ltd.), 02 July, 2002 (02.07.02), (Family: none) | 8,9,45,46 |
| Y | JP 2002-212333 A (Nikkiso Co., Ltd.), 31 July, 2002 (31.07.02), (Family: none) | 8,9,45,46 |
| Y | JP 2000-107577 A (Teijin Ltd.), 18 April, 2000 (18.04.00), (Family: none) | 8,45 |
| A | JP 2001-333974 A (Director General of National Institute of Advanced Industrial Science and Technology), 04 December, 2001 (04.12.01), (Family: none) | 1-37,41-71 |

Form PCT/ISA/210 (continuation of second sheet) (July 1998)

**INTERNATIONAL SEARCH REPORT**

International application No.

PCT/JP03/10692

---

| Box I | Observations where certain claims were found unsearchable (Continuation of item 2 of first sheet) |

This international search report has not been established in respect of certain claims under Article 17(2)(a) for the following reasons:

1. [×] Claims Nos.: 38-40, 72-77

    because they relate to subject matter not required to be searched by this Authority, namely:
    The inventions as claimed in the above claims pertain to methods for treatment of the human body by therapy or diagnostic methods.

2. [ ] Claims Nos.:

    because they relate to parts of the international application that do not comply with the prescribed requirements to such an extent that no meaningful international search can be carried out, specifically:

3. [ ] Claims Nos.:

    because they are dependent claims and are not drafted in accordance with the second and third sentences of Rule 6.4(a).

---

| Box II | Observations where unity of invention is lacking (Continuation of item 3 of first sheet) |

This International Searching Authority found multiple inventions in this international application, as follows:
    (See extra sheet.)

1. [×] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**   [ ]   The additional search fees were accompanied by the applicant's protest.

[×]   No protest accompanied the payment of additional search fees.

---

Form PCT/ISA/210 (continuation of first sheet (1)) (July 1998)

**INTERNATIONAL SEARCH REPORT**

International application No.

PCT/JP03/10692

Continuation of Box No.II of continuation of first sheet(1)

Referential document:
1. International Patent Publication No.513073/2001

According to the statement in the description of the present case, it is recognized that the invention as claimed in claim 1 relates to a fibrin-containing biological scaffold material to be used in the case of employing a fibrin composition for the regeneration of a human tissue and cell proliferation, characterized by containing a mixture of a fibrinogen concentrate, which is obtained from human plasma by a quick and rough purification method, with a fibrinogen activator. Thus, it appears that there is a technical relationship to the inventions as claimed in claims 2 to 40 involving "a special technical feature", i.e., being a fibrin-containing biological scaffold material containing a mixture of a fibrinogen concentrate with a fibrinogen activator.

On the other hand, it is recognized that the inventions as claimed in claims 41 to 77 relate to a system for concentrating fibrinogen from human plasma with the use of a plasma component fractionating membrane to obtain a fibrin-containing biological scaffold material, a method of operating this system and a process for producing a fibrin-containing biological scaffold material by using a concentration system with the use of a plasma component fractionating membrane. However, it cannot be said from the following two reasons that there is any technical relationship involving the same "special technical feature" between the inventions as claimed in claims 1 to 40 and the inventions as claimed in claims 41 to 77. (The term "special technical feature" as used herein means a technical feature that defines a contribution which each of the claimed inventions, considered as a whole, makes over the prior art. See, if needed, Rule 13.2 of the regulations under the Patent Cooperation Treaty.)

(1) It cannot be recognized that such a fibrin-containing biological scaffold material contains a mixture of a fibrinogen concentrate with a fibrin activator.

(2) Although the inventions as claimed in claims 41 to 77 have a common constitution of concentrating fibrinogen from human plasma, it is described in the document 1 to extract fibrinogen from human plasma.

Such being the case, it is concluded that the present application has 2 groups of inventions, i.e., the inventions as claimed in claims 1 to 40 and the inventions as claimed in claims 41 to 77.

Form PCT/ISA/210 (extra sheet) (July 1998)